(19) Europäisches Patentamt
European Patent Office
Office européen des brevets

(11) **EP 3 149 541 B1**

(12) **EUROPÄISCHE PATENTSCHRIFT**

(45) Veröffentlichungstag und Bekanntmachung des Hinweises auf die Patenterteilung:
**05.07.2023 Patentblatt 2023/27**

(21) Anmeldenummer: **15727609.8**

(22) Anmeldetag: **28.05.2015**

(51) Internationale Patentklassifikation (IPC):
**A63B 71/06** (2006.01)   **G02C 7/10** (2006.01)
**A61F 9/02** (2006.01)

(52) Gemeinsame Patentklassifikation (CPC):
**G02C 7/101; A61F 9/023;** A63B 2071/0666

(86) Internationale Anmeldenummer:
**PCT/EP2015/061918**

(87) Internationale Veröffentlichungsnummer:
**WO 2015/181340 (03.12.2015 Gazette 2015/48)**

(54) **ELEKTRONISCHE BRILLE**

ELECTRONIC SPECTACLES

LUNETTES ÉLECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.05.2014 DE 102014107587**
**11.06.2014 DE 102014108190**

(43) Veröffentlichungstag der Anmeldung:
**05.04.2017 Patentblatt 2017/14**

(73) Patentinhaber: **Inoptec Limited**
**85406 Zolling (DE)**

(72) Erfinder: **KNOLL, Ralf G. J.**
**85406 Zolling (DE)**

(74) Vertreter: **Köllner, Malte**
**Köllner & Partner mbB**
**Patentanwälte**
**Vogelweidstraße 8**
**60596 Frankfurt am Main (DE)**

(56) Entgegenhaltungen:

| | |
|---|---|
| EP-A2- 1 275 556 | DE-A1- 2 001 086 |
| DE-A1-102012 217 326 | FR-A1- 2 988 333 |
| GB-A- 2 420 183 | GB-A- 2 445 365 |
| JP-A- H0 882 751 | US-A- 5 305 012 |
| US-A- 5 654 786 | US-A1- 2004 012 762 |
| US-A1- 2013 194 244 | US-A1- 2013 293 379 |
| US-B1- 7 970 172 | |

• Philip Garvey ET AL: "Countermeasures for Reducing the Effects of Headlight Glare", , 1. Dezember 2001 (2001-12-01), Seiten 1-110, XP055207610, Gefunden im Internet: URL:http://www.lightmare.org/docs/AAAFoundationHeadlightGlare2001.pdf [gefunden am 2015-08-13]

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Gebiet der Erfindung

Die Erfindung betrifft eine elektronische Brille und ein Verfahren zur Blendunterdrückung, d.h. zum Regeln der Helligkeit des auf mindestens ein Auge treffenden sichtbaren Lichts.

Stand der Technik

Die durch einen Lichtmodulator hindurchtretende Lichtintensität lässt sich mit Hilfe von diversen am Markt verfügbaren Flüssigkristall-Zellen (TN, STN, Fe-LC, etc.) derart elektrisch steuern, dass mindestens 2 Zustände erreicht werden, nämlich durchlässig transparent - oder undurchlässig dunkel - wie es bei gängigen aktiven 3D-Fernseh- oder Kinobrillen der Fall ist (sog. Shutter-Brillen).

Gemäß dieser Grundidee wurden schon in den 60iger Jahren erste Versuche zu "elektronischen Sonnenbrillen" unternommen, um dem Träger einer solchen Brille eine variable Transmission zu bieten.

Einige bekannte elektronische Sonnenbrillen arbeiten mit einer reinen Steuerungen (statt einer Regelung), d.h. die Fotosensoren liegen an der Außenseite der Brille, so dass lediglich die Helligkeit gemessen wird, die auf die Brille von außen auftrifft (s. z. B US 5,172,256 A oder DE 10 2012 217 326 A1). Demensprechend wird mittels einer Kennlinie, die nur auf reinen Erfahrungswerten beruht, ein LCD entsprechend hell oder dunkel geschaltet.

Zudem existieren oftmals viel zu wenige Sensoren, deren Empfangsrichtung zudem unspezifisch ist (die Sensoren zeigen nach vorn oder in Richtung Himmel). Dies führt sehr oft zu völlig falschen und sogar gegenteiligen Reaktionen der Brille. Beispiel: Schaut der Träger der Brille beispielsweise in eine dunkles Bertachtungsgebiet (dunkle Ecke), während aber gleichzeitig die Brille von einem verirrten Strahl Sonnenlicht erfasst wird (durch zufällige Reflexionen an Gegenständen oder bewegte Blätter im Wald, die ein feines, variables Hell-Dunkel-Muster werfen), so wird das LC dunkel obwohl es eigentlich hell werden sollte, weil der Träger den dunklen Bereich sehen will.

Elektronische Systeme zur Unterdrückung von Blendlicht mit dem Ziel der Sichtverbesserung gibt es schon seit mehr als 80 Jahren (siehe z.B. US 2,066,680 A). In diesem Patent von 1934 wird das Licht der eigenen Scheinwerfer mit Hilfe rotierender mechanischer Schlitz- oder Fächerscheiben ("Chopper") in ein rechteckförmiges Signal (längs der Zeitachse) moduliert, während eine völlig identische Schlitz- oder Fächerscheibe in Blickrichtung vor dem Gesichtsfeld des Nutzers (Visor) exakt das gleiche vollbringt: d.h. mit exakt gleicher Frequenz und Phasenlage, wird synchron zum modulierten Scheinwerferlicht die Außenwelt vom Nutzer wahrgenommen.

Ist das Visor, durch welches der Nutzer blickt, beispielsweise 50% der Zeit geschlossen (Puls-Pause-Verhältnis = 1:1), so wird 50% des unerwünschten Lichtes (z.B. tief stehende Sonne) unterdrückt, und somit die Sichtbarkeit der zu betrachtenden Gegenstände erhöht.

Später ersetzten elektronisch ansteuerbare Lichtmodulatoren die mechanischen Lichtmodulatoren, insbesondere in Form von Flüssigkristallzellen, und auch die Lichtquellen wurden zunehmend schneller und einfacher elektronisch ansteuerbar (siehe z.B. DE 101 34 770 A1, DE 2 001 086 A, WO 2013/143 998 A2).

Aufgabe

Aufgabe der Erfindung ist es, eine Brille und ein Verfahren anzugeben, die für Sichtverbesserungen des Brillenträgers unter unterschiedlichen Bedingungen sorgen.

Lösung

Diese Aufgabe wird durch die Gegenstände der unabhängigen Ansprüche gelöst. Vorteilhafte Weiterbildungen der Gegenstände der unabhängigen Ansprüche sind in den Unteransprüchen gekennzeichnet. Der Wortlaut sämtlicher Ansprüche wird hiermit durch Bezugnahme zum Inhalt dieser Beschreibung gemacht.

Zur Lösung der Aufgabe wird eine Brille für einen Träger mit mindestens einem Auge vorgeschlagen. Die Brille hat mindestens ein Brillenglas, wobei das mindestens eine Brillenglas eine Flüssigkristallzelle aufweist, deren Transmission durch eine geeignete Ansteuerung veränderbar ist. Ferner weist die Brille einen Eye-Tracker auf, der die Blickrichtung des Auges bestimmt. Ferner mindestens einen Sensor zum Messen der Helligkeit des auf ihn treffenden sichtbaren Lichts, wobei der Sensor augenseitig des Brillenglases angeordnet ist, die Helligkeit durch das mindestens eine Brillenglas misst, und

- ein abbildendes System mit einer Kamera oder
- mindestens 3 Sensoren, die ein Koordinatensystem aufspannen, oder
- ein Facettenauge

aufweist.

Ein elektronisches Facettenauge besteht aus vielen Einzelaugen, ähnlich zu dem in der Biologie verwendeten Begriff der "Ommatidien" in der Beschreibung eines Facettenauges von Fluginsekten, jedoch bestehend aus elektrischen Photosensoren, die wiederum am unteren Ende von lichtleitenden Trichtern (ohne Linse) sitzen - oder mit jeweils einer vorgeschalteten Mikro-Linse versehen sind - oder einer Kombination aus beiden (Trichter und Mikro-Linse) (siehe z.B. EP 0813079 A2).

Der mindestens eine Sensor bestimmt die Helligkeit des aus der mit dem Eye-Tracker bestimmten Blickrichtung des Auges auf ihn treffenden sichtbaren Lichts.

Die Brille verfügt ferner über einen geschlossenen Regelkreis, der die Transmission der Flüssigkristallzelle regelt, wobei ein Sollwert für die Helligkeit am Auge vorgegeben ist, und wobei der Regelkreis als Istwert die vom Sensor gemessene Helligkeit in Blickrichtung des Auges heranzieht.

Mit einer solchen Brille lässt sich die Helligkeit schnell und exakt an die Blendung aus der tatsächlichen Blickrichtung des Brillenträgers anpassen, etwa wenn einem Autofahrer ein anderes Auto entgegenkommt oder wenn ein Autofahrer an einem sonnigen Tag in einen Tunnel fährt oder aus einem Tunnel heraus fährt.

Erst jetzt, im Zuge von extremer Miniaturisierung und "tragbarer Elektronik" (wearable electronics), ist es möglich, mittels Miniatur-Elektronik, die sich auch in eine Brille leicht und einfach integrieren lässt, solche leistungsfähigen und sicheren Systeme zur Sichtverbesserung zu realisieren.

Um die Anwendungsmöglichkeiten der Brille zu erweitern, bietet es sich an, die Transmission der Flüssigkristallzelle der Brille nicht auf eine jeweils passende Graustufe einzustellen, sondern die Brille in kurzer Folge zwischen so weit wie möglich transmittierend und so weit als möglich Licht blockierend umzuschalten. Damit das menschliche Auge davon möglichst nichts sieht, sollte ein Zyklus (Periode) aus einer transmittierenden Zeitspanne und einer blockierenden Zeitspanne maximal ein Vierundzwanzigstel (1/24) einer Sekunde dauern.

Solche Systeme funktionieren besonders gut, wenn der Mensch die Regelung nicht mehr wahrnimmt, also mit Zykluszeiten oberhalb der Flimmer-Grenzfrequenz (Critical Flicker Frequency - CFF) von etwa 60Hz arbeiten.

Um dies erreichen zu können, sollte die Flüssigkristallzelle derart ausgebildet sein, dass sie ihre Transmission von 90% auf 10% und von 10% auf 90% in maximal 10 ms ändern kann.

Hat man eine solche Flüssigkristallzelle, kann die Transmission der Flüssigkristallzelle zwischen Zuständen hoher und niedriger Transmission umgeschaltet werden. Dazu müssen Mittel zum Regeln oder Steuern der Zeiten des Zustands hoher und niedriger Transmission der Flüssigkristallzelle sowie des Wechsels zwischen diesen beiden Zuständen vorhanden sein. Sinnvollerweise ist die Regelung oder Steuerung und der geschlossene Regelkreises derart ausgebildet, dass die Zeiten des Zustands hoher Transmission mit zunehmender Helligkeit des auf den mindestens einen Sensor treffenden sichtbaren Lichts kürzer werden (Pulsweiten-Modulation, PWM). Dabei findet der Wechsel zwischen den Zuständen hoher und niedriger Transmission mit einer zeitlichen Frequenz statt, die das menschliche Auge nicht auflösen kann.

Noch präziser und noch schonender für die Augen des Brillenträgers wird die Regelung, wenn der Regelkreis derart ausgebildet ist, dass er bei der Bestimmung der Helligkeit aus der Blickrichtung des Auges eine nutzerspezifische Augen/Netzhaut-Empfindlichkeitskurve zur Gewichtung der Helligkeit berücksichtigen kann.

Die nutzerspezifischen Augen/Netzhaut-Empfindlichkeitskurve berücksichtigt z.B. das Alter des Brillenträgers, sonstige allgemeine und/oder individuelle augenspezifische Parameter, vornehmlich bezüglich des Einfallswinkels ins Auge, aber auch bezüglich anderer lichttechnischer Größen die Einfluss auf die Wahrnehmung haben, wie z.B. Leuchtdichte, Entfernung der Lichtquelle bzw. Lichtintensität oder Lichtstärke (Lichtfluss pro Winkel Steradiant), Beleuchtungsstärke, deren jeweilige absolute Größen als Schwelle am Auge, Lichtfluss, Größe der Störquelle (Punkt vs. Fläche), Farbe oder Spektralverteilung der Quelle und deren zeitliche Variation, Voreinstellung des Auges (photopisches vs. skotopisches Sehen, etc.).

Diese Empfindlichkeitskurven können heuristisch und logisch ermittelt werden, werden aber in der Regel empirisch ermittelt, wie beispielsweise herangezogen und ausgewertet in der Arbeit: Douglas Mace, Philip Garvey, Richard J. Porter, Richard Schwab, Werner Adrian: "Counter-measures for Reducing the Effects of Headlight Glare"; Prepared for: The AAA Foundation for Traffic Safety, Washington, D.C., December 2001.

Die vorgenannten Empfindlichkeitskurven des menschlichen Auges sind als Gewichtungsfaktoren in verschiedenen Tabellen (Lookup Table - LUT) oder als berechenbare Formel hinterlegt - jedenfalls so, dass im geschlossenen Regelkreis des Systems, bestehend aus Innen-sensor, einem Micro-Controller und dem eingestellten Sollwert, diese Gewichtungsfaktoren in Echtzeit in das Stellsignal zum Einstellen des Transmission der Flüssigkristallzelle einbezogen werden.

Beispielsweise beschreibt eine Formel von Adrian und Bhanji (Adrian, W. und Bhanji, A. (1991). "Fundamentals of disability glare. A formula to describe stray light in the eye as a function of the glare angle and age." Proceedings of the First International Symposium on Glare, Orlando, Florida, pp. 185-194) zur Bestimmung der "unmöglichen Lesbarkeit und Erkennbarkeit von Objekten bei Gegenlicht" (Engl. "Disability Glare"), die Abhängigkeit vom Einfallswinkel des Störlichtes ins Auge, unter dem zunehmend nichts mehr erkennbar ist.

Beispiel: Fällt Störlicht direkt senkrecht in das Auge, so ist die Blendung am höchsten (Maximum in der Gewichtungsformel). Nachdem der Eye-Tracker die Blickrichtung bestimmt (Vektor ET(x.y,z)), und der Innensensor und/oder der Außensensor die Einfallsrichtung des Störlichtes (Vektor Glare(x,y,z)), so kann der Microcontroller prüfen, ob diese beide Vektoren kollinear sind, d.h. die gleiche Richtung aufweisen, und dementsprechend mit der vorgenannten Gewichtungskurve maximal bewerten. Ist die Gewichtungskurve beispielsweise als LUT hinterlegt, so bewegt sich diese mit Blickrichtungs-Vektor ET(x,y,z) virtuell im Speicher des Microcontrollers der Augenbewegung entsprechend hin und her. Ist sie als Formel hinterlegt, wird dementsprechend der Vektor in einen Winkel umgerechnet.

Dadurch muss man die Empfindlichkeitskurven nicht mehr als spezielle Vorsatzlinsen anfertigen (z.B. individuelle Freiform-Plastiklinse), die das Licht entsprechend "gewichten" bevor es auf einen Photosensor trifft. Jegliche gewichtende Vorsatzlinsen, oder gar bewegliche Vorsatzlinsen, die die Empfindlichkeit der Retina nachbilden, können entfallen, da alles rein in Software abgebildet ist, während alle Sensoren starr montiert sind.

Als besonders schonend für das Auge hat sich eine Festlegung des Sollwerts des Regelkreises auf eine mittlere Helligkeit im Bereich von 20 bis 400 lx (Lux) erwiesen. Ein solcher Wert erlaubt eine Regelung auf eine gleichbleibende Helligkeit für die Augen des Brillenträgers, wenn sich die äußere Helligkeit von sehr hell bis hinunter zu dem eingestellten Sollwert oder umgekehrt ändert, etwa wenn ein Auto an einem Sommertag in einen Tunnel einfährt oder aus einem Tunnel herausfährt. Die Änderungen der Helligkeit bzw. der Beleuchtungsstärke können in einem solchen Moment einen Faktor 1000 oder mehr ausmachen. Der Brillenträger ist diesen gegebenenfalls sehr schnellen Helligkeitsschwankungen nicht ausgesetzt. Sie werden von der Regelung der Brille stets nivellierend ausgeglichen.

Die Einfahrt in einen dunklen Tunnel oder dunklen Schattenbereich (Wald etc.) an einem hellen sonnigen Tag ist ein typischer Anwendungsfall. Da der hier eingestellte Sollwert tagsüber einer dunklen Sonnenbrille entspricht, ist das Auge stets von vornherein dunkel adaptiert und vorbereitet, so dass beim Eintritt in den Dunkelbereich das Brillenglas lediglich in Echtzeit transparenter und klarer (offener) geregelt werden muss, um sofort im Dunkeln sehen zu können. Die ohne diese Brille benötigte Dunkeladaptionszeit des menschlichen Auges von ca. 30 Sekunden wird somit auf einen Sekundenbruchteil (z.B. 10 Millisekunden) reduziert, so dass man sofort im Dunkeln sehen kann. Bei der Tunnelausfahrt zurück ins Helle gilt genau das Umgekehrte.

Weitere Regelungsmöglichkeiten, die weiter unten geschildert werden, eröffnen sich, wenn die Brille mindestens einen weiteren Helligkeits-Sensor aufweist, der auf der vom Auge abgewandten Seite der Brille angeordnet ist (Außensensor) und die Helligkeit des Umgebungslichts bestimmt.

Zum Beispiel kann dann der Sollwert des Regelkreises abhängig von der Helligkeit des Umgebungslichts verändert werden, wobei eine solche Veränderung des Sollwerts um mindestens einen Faktor zehn langsamer ist als die Regelung der Transmission der Flüssigkristallzelle erfolgen sollte, damit das Auge des Brillenträgers sich ohne Schwierigkeiten an dieser Änderung adaptieren kann.

Auf plötzliche Helligkeit-Veränderungen sollte die Brille innerhalb von 10 $\mu$s bis einer Sekunde derart reagieren, dass die Flüssigkristallzelle (LC) auf den Zustand niedriger Transmission eingestellt wird.

Die Brille wird in extremen Situationen, bei sogenannter "Disability Glare", bei der (siehe oben) ein Mensch nichts mehr lesen oder sehen kann, nämlich dann, wenn exakt senkrecht ins Auge (unter null Grad) eine extrem starke Blendung auftritt, wie beim direkten Blick in die Sonne, komplett geschlossen, d.h. sie wird auf völlig schwarz eingestellt.

Eine solche Regelung ist insofern nicht kritisch, als dass es keine Rolle spielt, ob man nichts sieht wegen extremer Blendung oder weil die Brille schwarz abdunkelt, jedoch hat letzterer Zustand den Vorteil dass das Auge geschützt und dunkeladaptiert bleibt.

Nach einer gewissen Zeit bzw. einer Blickrichtungsänderung des Brillenträgers wird die Brille anschließend langsam wieder Licht transportieren lassen.

Noch präziser wird die Regelung der Blendunterdrückung, wenn die Brille zwei Brillengläser für zwei Augen eines Brillenträgers aufweist, sowie je einen augenseitigen Sensor für jedes Brillenglas zum Messen der Helligkeit des auf das jeweilige Auge treffenden sichtbaren Lichts. Mithilfe je eines Regelkreises für jedes Brillenglas kann man dann die Regelung für jedes Auge individuell erfolgen.

Eine Verstärkung des Helligkeits-/Kontrast-Umfangs kann mit einer derartigen Brille erreicht werden, wenn die Sollwerte für die beiden Augen um 1% bis 60% voneinander abweichen. Typische Werte für die Rechts-Links-Abweichungen in der Praxis liegen bei 5% - 30%. In Analogie zur High Dynamik Range (HDR) Fotografie kann hier von "HDR-Sehen" gesprochen werden.

Solche Systeme standen bis dato nur theoretisch zur Verfügung, aber erst jetzt, durch die Verfügbarkeit von extrem schnellen Modulatoren und sehr schnellen Prozessoren können intelligente und sicherheitsrelevante Mehrkanal-Echtzeit-Regelungen zur Sichtverbesserung realisiert werden, wobei das linke und rechte Auge getrennt behandelt werden, und/oder mehrere Nutzer für Gruppenanwendungen einbezogen werden können.

Um dies zu gewährleisten, sollte der Regelkreis derart ausgebildet sein, dass bei der Regelung der Helligkeit des auf ein Auge treffenden sichtbaren Lichts die Regelung der Helligkeit für das andere Auge berücksichtigt werden.

Die Brille kann auch mit einer Lichtquelle kombiniert werden, die an der vom Auge abgewandten Seite der Brille angeordnet ist. Sinnvollerweise wird dann die Lichtquelle abhängig von der Blickrichtung des Brillenträgers gesteuert. Auf diese Weise kann der Verdunkelung entgegengewirkt werden, die durch das Shuttern der Brille zur Vermeidung von Blendung hervorgerufen wird. Denkbar sind zum Beispiel vier LEDs, an jeder Brillenecke eine.

Der Eye-Tracker stellt dann fest, welche von den vier LEDs je nach Blickrichtung bevorzugt mit Energie versorgt werden soll - entweder nur eine in Blickrichtung korrespondierende LED bei Blick nach außen oben/unten - oder zwei in Blickrichtung korrespondierende LEDs bei Blick seitlich mittig - oder alle vier LED bei Blick geradeaus nach vorn.

Weitere Möglichkeiten:

Anstelle von oder zusätzlich zu den vier starr montierten LEDs an den Ecken einer Brille, können auch beliebige andere Lichtquellen/Scheinwerfer mit Hilfe des Eye-Trackers in ihrer Leuchtrichtung in die Blickrichtung gesteuert werden.

Zu diesem Zweck sind diese Lampen elektro-mechanisch schwenkbar, ähnlich wie beim elektronisch einschwenkenden Kurvenlicht bei Kraftfahrzeugen oder bei schwenkbaren 3-Achsen-Überwachungskameras oder bei frei beweglichen von Hand getragenen Systemen, die aber via elektronischer oder massenträger kardanischer Aufhängung (Engl. Gimbal oder Steadycam-Verfahren) ein eigenes stabiles Koordinatensystem bezüglich Erde oder Träger aufrecht erhalten und in dessen Relation der Scheinwerfer dann in Blickrichtung schwenken kann.

Somit kommen alle Arten von LED-Scheinwerfern auf allen Arten von Halterungen in Frage: Auto, Helm, Fahrrad, Motorrad, Hand, Schulter, Körper, Gewehr usw..

Besonders effektiv ist dies, wenn die Leuchtzeiten und die Leuchtintensität der Lichtquelle derart geregelt werden, dass die Lichtquelle während der Zeiten des Zustands hoher Transmission der Flüssigkristallzelle leuchtet. Dabei sollte das zeitliche Integral des Produkts aus der Leuchtintensität der Lichtquelle und der Transmission der Flüssigkristallzelle bei einer Veränderung der Zeiten des Zustands hoher Transmission innerhalb einer vorgegebenen Toleranz konstant bleiben.

Eine solche blitzende Lichtquelle kann beispielsweise ein Autoscheinwerfer sein, der für den Autofahrer die Straße und das Umfeld stets mit konstanter Helligkeit beleuchtet, während die Blendung durch entgegenkommende Fahrzeuge durch das Shuttern der Brille effektiv verhindert wird. Aber auch andere Arten von Scheinwerfern, wie Fahrradlampen, Helmlampen, Taschenlampen, sind in dem hier beschriebenen Sinne einsetzbar.

Da die von einem Außenstehenden oder entgegenkommenden Fahrzeug wahrgenommene Helligkeit des Autoscheinwerfers unter diesen Bedingungen stets konstant ist, unabhängig davon, wie das Puls-Pausen-Verhältnis geregelt ist, kann ein solcher Autoscheinwerfer problemlos im Sinne einer Ersatz-Strategie (*replacement strategy*) oder ein Hinzukaufen von Zusatzscheinwerfern im Sinne einer Zubehör-Strategie (*special accessories strategy*) angeboten werden.

Erst jetzt ist es möglich, mittels leistungsstarker Weißlicht- und/oder RGB-LED/LASER, solche leistungsfähigen und sicheren Systeme zur Sichtverbesserung zu realisieren.

Neben einem Autoscheinwerfer sind insbesondere auch

- eine Lichtquelle zum Blenden eines Lebewesens, eines optischen Sensors oder einer Kamera, und/oder
- eine Anzeige an der vom Auge abgewandten Seite des Brillenglases und/oder
- eine Anzeige augenseitig des Brillenglases und/oder
- ein Head-Up-Display

als Lichtquellen denkbar.

Als Anzeigen an der vom Auge abgewandten Seite des Brillenglases kommen beispielsweise ein Smartphone, Tablett, Laptop, Cockpit-Anzeige, etc. in Betracht.

Als Anzeigen augenseitig des Brillenglases kommen zum Beispiel "Google Glass" oder Anzeigen zur virtuellen oder erweiterte Realität (*"augmented reality"*) in Betracht.

Unter Head-up-Displays (HUD) werden unterschiedliche Anzeigen subsummiert, einige davon augenseitig der Brille, einige außerhalb der Brille, etwa in Form eines Helms mit Display. Allen gemeinsam ist, dass man durch sie hindurch schauen kann, das Head-up-Display jedoch zusätzliche Informationen eingeblendet.

Alle diese Anzeigen werden auf die geschilderte Weise gegen die Sonne oder sonstige störende Blendlichtquellen ablesbar.

Im Folgenden werden einzelne Verfahrensschritte näher beschrieben. Die Schritte müssen nicht notwendigerweise in der angegebenen Reihenfolge durchgeführt werden, und das zu schildernde Verfahren kann auch weitere, nicht genannte Schritte aufweisen.

Die Aufgabe wird außerdem gelöst durch ein Verfahren zum Regeln der Helligkeit des auf mindestens ein Auge treffenden sichtbaren Lichts, mit folgenden Schritten:

1. eine Brille wird zur Verfügung gestellt, wobei die Brille folgendes aufweist:

1.1 mindestens ein Brillenglas;

1.1.1 wobei das mindestens eine Brillenglas eine Flüssigkristallzelle (LC) aufweist, deren Transmission (TR) durch eine geeignete Ansteuerung veränderbar ist;

1.2 einen Eye-Tracker (ET), der die Blickrichtung des Auges bestimmt;

1.3 mindestens einen Sensor (IL, IR) zum Messen der Helligkeit des auf den Sensor treffenden sichtbaren Lichts;

1.3.1 wobei der mindestens eine Sensor (IL, IR) augenseitig des Brillenglases angeordnet ist;

1.3.2 wobei der mindestens eine Sensor (IL, IR) die Helligkeit durch das mindestens eine Brillenglas misst;

1.3.3. wobei der mindestens eine Sensor (IL, IR)

1.3.3.1 ein abbildendes System mit einer Kamera oder

1.3.3.2 mindestens drei Sensoren, die ein Koordinatensystem aufspannen oder

1.3.3.3 ein Facettenauge aufweist;

1.3.4 wobei der mindestens eine Sensor (IL, IR) die Helligkeit des aus der mit dem Eye-Tracker (ET) bestimmten Blickrichtung des Auges auf ihn treffenden sichtbaren Lichts bestimmt;

2. die Transmission der Flüssigkristallzelle (LC) wird mittels eines geschlossenen Regelkreises (MC) geregelt;

2.1 wobei ein Sollwert für die Helligkeit am Auge vorgegeben ist;

2.2 wobei der Regelkreis als Istwert die vom Sensor gemessene Helligkeit in Blickrichtung des Auges heranzieht.

Weitere Einzelheiten und Merkmale ergeben sich aus der nachfolgenden Beschreibung von bevorzugten Ausführungsbeispielen in Verbindung mit den Unteransprüchen. Hierbei können die jeweiligen Merkmale für sich alleine oder zu mehreren in Kombination miteinander verwirklicht sein. Die Möglichkeiten, die Aufgabe zu lösen, sind nicht auf die Ausführungsbeispiele beschränkt. So umfassen beispielsweise Bereichsangaben stets alle - nicht genannten - Zwischenwerte und alle denkbaren Teilintervalle.

Intelligente Brille mit Eye Tracker

Alle oben genannten Probleme werden mit einer "intelligenten Brille" gelöst, bestehend aus mindestens einem Brillenglas, das durch eine Flüssigkristallzelle LC realisiert ist, mit einem Closed-Loop-Echtzeit-PID-Regelkreis, vorzugsweise jedoch bestehend aus zwei völlig unabhängigen Brillengläsern und Regelkreisen des genannten Typs. Die Transmission der Flüssigkristallzelle ist durch geeignete Ansteuerung derart veränderbar, dass sie zwischen Zuständen hoher und niedriger Transmission umgeschaltet werden kann, um hierdurch einen Shutter-Effekt zu erzielen. Erfolgt dieser schnell genug, kann der Sichteindruck des jeweiligen Auges verändert werden, wobei die Trägheit der visuellen Wahrnehmung des Menschen ausgenutzt wird.

Damit sich ein Closed-Loop Regelkreis (geschlossener Regelkreis) realisieren lässt, muss pro Auge mindestens ein Fotosensor "innenliegend" sein, nämlich derart, dass er in Blickrichtung, gleichsam wie das menschliche Auge durch den Shutter hindurchschaut und somit die "tatsächliche Helligkeit" misst. Diese dient für die Regelung als "Istwert".

Definitorische Anmerkung zum vorgenannten Helligkeits-Istwert, der durch den Shutter hindurch gemessen wird, weil gegebenenfalls je nach technischer Sachverhaltsbeschreibung zwischen einem diskreten (punktuellen) Istwert auf der Zeitachse und einem Integrationsergebnis über einen kompletten Shutter-Zyklus T unterschieden werden muss:

1. Genau genommen können die heutzutage verfügbaren Fotosensoren so schnell ausgelesen werden, dass durch den Shutter hindurchtretende Lichtintensitäten auf der Zeitachse punktuell (z.B. mit Abtastfrequenzen im Mikrosekundenbereich) gemessen werden können, ähnlich wie bei einem digitalen Speicher-Oszilloskop mit optischem Messkopf, so dass ein diskreter Istwert-Verlauf in einem flüchtigen Speicher des Mikrocontrollers hinterlegt werden kann. In diesem Verlauf lässt sich genau erkennen, wann der Shutter innerhalb eines Pulsweitenmodulations(PWM)-Zyklus T jeweils geöffnet ($T_{on}$ bzw. transparent) und wann er geschlossen ($T_{off}$ bzw. intransparent) ist. Läuft das Shuttersystem beispielsweise mit einer Grundfrequenz von 100 Hz, so beträgt die zeitliche Speichertiefe 1/100 Hz = 10 ms. Der Mikrocontroller kann am Ende eines Zyklus rein mathematisch ein Integral über diesen Helligkeitsverlauf bilden und somit den "Istwert" eines Zyklus liefern.

2. Andererseits könnte derselbe Fotosensor ebenso über den gesamten Zyklus T, also über die vorgenannten 10 Millisekunden, physikalisch und elektronisch bzw. schaltungstechnisch bedingt derart integrieren, dass exakt am Ende des Zyklus T ein Messergebnis vorliegt, welches dann vom Mikrokontroller ausgelesen wird, ohne dass dieser eine mathematische Mittelung leisten muss. In der hier vorliegenden Erfindung wird zur Messung des Istwertes ein Fotosensor verwendet, der die schnelle punktuelle / diskrete Messung ermöglicht. Um Missverständnisse zu vermeiden, wird vorliegenden im Text in der Regel der Begriff "Istwert" verwendet, wenn ein über die Zykluszeit T umgerechneter oder integrierter "Grauwert" (mittlere durchtretende Helligkeit im Zyklus T) gemeint ist - zumal der Mensch ebenfalls nur Grauwerte wahrnimmt, auch wenn in Wirklichkeit nur zeitliche Verhältnisse von $T_{on}$ zu $T_{off}$ durchgelassen werden.

Der Fotosensor nimmt damit quasi die Rolle des Auges ein, misst also stellvertretend für das Auge die "echte Helligkeit", die in das Auge fällt, nicht nur irgendeine zufällig äußere Helligkeit. Das Auge wird bei einer ON-OFF-Keying-PWM insofern als Tiefpass benutzt, als dass nur im Auge bzw. nur in der menschlichen Wahrnehmung die Grauwerte entstehen, während die Brillengläser aber in Wirklichkeit niemals Grauwerte annehmen. In Analogie zu den oben aufgeführten Integrations-Szenarien zum Istwert (1. und 2.) kann streng genommen noch ein drittes Szenario definiert werden, indem der Mikrocontroller und/oder der Fotosensor so lange integriert, bis ein Grauwert erreicht wird, der auch vom Menschen tatsächlich als Grauwert wahrgenommen werden kann (z.B. nach Integration über etwa 250 bis 500 Millisekunden). Sollte dieser wahrnehmbare Istwert gemeint sein, so wird dies im Text in der Regel gesondert kenntlich gemacht.

Der Fotosensor bzw. Helligkeitssensor hat einen gewissen Abstand (typischerweise 1-3 mm) zur LC-Zelle, so dass aufgrund seines Öffnungswinkels die effektiv betrachtete LC-Fläche größer ist als die Chipfläche des Sensors. Dies führt zu einer besseren Mittelung der Helligkeit und einer genaueren/stabileren Messung im Falle von punktueller "LC-Domainbildung", oder im Falle von punktueller Verschmutzung auf der Gegenseite der LC-Zelle. Aus Sicherheitsgründen und aus thermischen Gründen ist es ohnehin zweckmäßig, ein äußeres Schutzglas, welches auch das äußere Design der Brille ausmacht, in einem Abstand von 1-3 mm vor der LC-Zelle anzubringen. Damit haben solche punktuellen Verschmutzungen (kleine Fliegen, Staubkörner, etc.) keinen Einfluss auf das LC und schon gar keinen Einfluss auf den Fotosensor mehr. Zudem werden die innenliegenden Fotosensoren (sofern es sich um klassische, also nicht transparente, Fotosensoren handelt) im äußeren LC-Randbereich bzw. Brillen-Rahmenbereich angebracht, so dass diese nicht das Sehfeld stören.

Um aber den Helligkeits-Istwert in der Mitte des LC-Shutters oder genauer, in der statistischen Ortsmitte der Pupille bei Geradeaussicht, möglichst genau zu bestimmen zu können, werden mindestens zwei, besser drei Fotosensoren pro Auge eingesetzt, deren Positionierung ein Koordinatensystem aufspannt. Beispielsweise können sie in einem Dreieckangeordnet sein, auf dessen Ecke der statistische Ortsmittelwert der Pupille zu liegen kommt, der in der Regel (d.h. bei nicht schielenden Menschen) identisch ist mit dem Punkt der Geradeaussicht. Mit Hilfe einer Triangulations-Berechnung lässt sich dann die mittlere Helligkeit in Bezug auf diesen statistischen Ortsmittelwert bzw. die Geradeaussicht berechnen und als "Istwert" für die Regelung heranziehen.

Zudem haben mehrere innenliegende Fotosensoren pro Auge den Vorteil, dass durch diese Redundanz die Mess-Sicherheit erhalten bleibt, auch bei Verschmutzung oder bei verstärktem punktuellem Lichteinfall (z.B. zufällige helle Reflexion auf nur einen von drei Fotosensoren).

Für die Regelung wird ein "Sollwert" benötigt, der zunächst mit einer Art Potentiometer oder vergleichbarem "Einsteller" so vorgegeben wird, dass das Auge konstant dunkeladaptiert bleibt, ähnlich wie bei einer relativ starken Sonnenbrille, z.B. mit Schutzstufe III (S3, 8-18% Transmission).

Der Regelkreis muss derart schnell sein, dass der Regelvorgang vom menschlichen Auge nicht mehr wahrgenommen werden kann, so dass die am Auge eintreffende Helligkeit stets konstant ist (dem Sollwert entsprechend), egal wie sich außen die Helligkeit ändert.

Es handelt sich also um einen sogenannten Echtzeit-Regelkreis, bei dem im eingeschwungenen Zustand (richtige PID-Parametrierung) das sogenannte Delta (Regelabweichung) - also die Differenz zwischen Sollwert und Istwert - stets Null ist.

Eine solche Regelung funktioniert jedoch nur, wenn die Brille absolut lichtdicht in Bezug auf Licht von außen ist. Das Brillengehäuse ähnelt daher einer Taucherbrille, Skibrille oder einer eng anliegenden Arbeitsschutzbrille mit weichen staub- und lichtdichten Augenmuscheln im Stile einer Schwimmbrille oder einer großen Brille mit breiten Seitenbügeln und Lichtschutz von oben und unten. Mit Hilfe eines elektrischen Potentiometers oder eines ähnlichen Einstellers lässt sich die Pupille des Trägers der Brille a) langsam öffnen, sogar regelrecht "aufdrehen", bis diese zu 75% über Normaldurchmesser bei Tageslicht geöffnet ist; und b) aufgrund der Echtzeitregelung konstant auf diesem Durchmesser halten, so dass sie quasi "ruhig gestellt" wird, egal wie sich die Helligkeit draußen ändern mag.

Dies geschieht für jedes Auge getrennt, wenngleich in der Startroutine zunächst jedes Auge den gleichen Sollwert eingestellt bekommen kann (z.B. 100 Lux am rechten (R) und linken (L) Auge). In der Praxis werden die Sollwerte R und L vergleichsweise langsam geändert (z.B. 2- bis 100-mal langsamer als die Helligkeitsregelung) und auch bewusst mit leichten Differenzen beaufschlagt (z.B. links 10% mehr Transparenz und rechts 10% weniger Transparenz). Die Gründe dafür werden nachfolgend erklärt.

Mindestens ein Außensensor pro Auge (OL, OR) erfasst grob und vergleichsweise langsam (z.B. binnen 1-2 Sekunden) die Tageslichtsituation im zeitlichen Mittel und stellt fest, ob ein heller Tag, bedeckter Tag oder eine Innenraumsituation vorliegt. Dies ist notwendig, weil der Dynamikumfang bezüglich der Beleuchtungsstärke tagsüber einen Bereich von 100 Lux bis 100.000 Lux umfasst, also etwa einen Faktor 10.000, während eine einfache LC-Zelle nur einen Faktor 1000 bis 5000 (Kontrastverhältnis) umfasst. Durch einen veränderlichen Sollwert, der vom Außensensor bestimmt wird (heller Tag, bedeckter Tag, ...) wird daher der "Arbeitspunkt" der LC Zelle während einer Initialisierungsroutine beim Einschalten in den richtigen Bereich geschoben (z.B. an einem sehr hellen Tag von initial 100 Lux am Auge auf 300 Lux am Auge).

Dieser vom Außensensor initiierte Sollwert wird auch dann schnell und dynamisch geändert, wenn der Regler am unteren oder oberen Anschlag ist, also die Regelabweichung nicht mehr Null sein kann, weil die Regelgröße an der LC-Zelle bzw. die Transmission einen nicht mehr steigerbaren Wert erreicht hat (d.h. ganz auf oder zu).

Dies sollte nicht regelmäßig der Fall sein, da vorgesehen ist, das Auge permanent dunkeladaptiert zu halten. Wenn sich die Lichtsituation insgesamt verändert, kann jedoch unter Berücksichtigung von elektronisch hinterlegten Erfahrungswerten sowie Informationen von den Außen- und Innensensoren, kurz vor Erreichen des Regler-Anschlags in eine bestimmte Richtung (LC ganz zu oder ganz auf) der Sollwert so geändert werden, dass der Regler weiterhin im "Regelbetrieb" bleibt und diesen Anschlag nicht wirklich erreicht, d.h. sich im weitesten Sinne logarithmisch oder ähnlich nichtlinear verhält - aber aufgrund der erhöhten durchtretenden Helligkeit (z.B. beim direkten Blick in die Sonne) ein sanftes und kontrolliertes Schließen der Iris ermöglicht. Diese Nachregelung des Sollwerts zur Erweiterung des Dynamikumfangs sollte allerdings nur in seltenen Ausnahmefällen auftreten; im Normalbetrieb wird die Pupille auf einen festen relativ dunklen Wert eingestellt (z.B. 75% über Normaldurchmesser), damit das bereits dunkeladaptierte Auge bei Eintritt in einen dunklen Raum sofort (d.h. z.B. innerhalb einer Millisekunde) zur Verfügung steht.

Zudem können die beiden Sollwerte (L und R) leichte Differenzen aufweisen, z.B. 5% bis 30% mehr Transparenz links als rechts, damit das Gehirn aus den beiden leicht unterschiedlichen Bildern in der Wahrnehmung unmerklich wieder ein Bild mit höherem Kontrastumfang (Dynamikbereich) machen kann (aus der Fotografie als HDR = "high dynamic range" bekannt - zwei unterschiedlich belichtete Fotos werde ineinander kopiert). Voraussetzung ist, dass der Kontrastunterschied nicht zu extrem wird, also für den Menschen unmerklich bleibt, z.B. 1% bis 60%, bevorzugt 5% bis 30%. Nicht ausgeschlossen werden auch höhere Werte > 30%, die aber dann zeitlich kürzer eingeblendet werden, derart, dass das Gehirn daraus trotzdem unmerklich ein neues Bild mit höherem Kontrastumfang konstruieren kann. Hierbei wird also mittels intelligenter Software-Algorithmen in die menschliche Wahrnehmung eingegriffen.

Zudem können in der Brille, wie heutzutage bei sog. "wearable Technology" und Smartphones üblich, auch Neigungs- und Beschleunigungssensoren integriert sein, so dass z.B. bei schneller Fahrt solche Helligkeits-Differenzen automatisch reduziert oder sogar abgeschaltet werden können, um beispielsweise unerwünschte Effekte zu vermeiden (z.B. Pulfrich-Effekt oder andere Wahrnehmungs-Artefakte).

Die höchste und komplexeste Form dieser Art von elektronischer Ansteuerung besteht in der Berücksichtigung der rechts-links kontralateralen Pupillenafferenz bei einer "Swinging-Flashlight-Test" (SWIFT) ähnlichen Beleuchtungssituation, die physiologisch über den überkreuzenden Links-Rechts-Nervensignalaustausch im Chiasma opticum und in nachfolgenden Teilen des Gehirns erfolgt. Konkret bedeutet dies, dass bei exakt gleich eingestellten elektronischen Sollwerten für beide Augen (L=R=const.) beim gesunden Menschen ohne Asymmetrien in der kontralateralen Pupillenafferenz (wie z.B. beim relativen afferenten Pupillendefekt RAPD) im Regelbetrieb keine neuronalen Reize über Kreuz ausgetauscht werden, da die Helligkeit auf beiden Augen stets konstant ist. Es gibt drei Arten, diesen Effekt auszunutzen:

1) Ein erhöhtes Stellsignal (z.B. verstärkte Abdunkelung) auf einem Kanal (L oder R), bei jeweils identischen Sollwerten (R=L=const.) signalisiert eine asymmetrische Beleuchtungssituation, z.B. übermäßig viel Außenlicht auf dem betreffenden Kanal. Der Mikrocontroller dieses Kanals teilt dem anderen Mikrocontroller oder der State-Machine des anderen Kanals das nahezu Erreichen oder Übersteuern des Kanals mit. Die unterbelichtete Seite kann dann aufmachen.

2) Absichtliches Betreiben in dem HDR-Differenzmodus kann dazu führen, dass derjenige Kanal, der heller (transparenter) geschaltet wird, insbesondere wenn dieser zu schnell und zu umfänglich transparent geschaltet wird (delta t, delta T relativ hoch), eine kontralaterale Pupillenkontraktion auf dem anderen Kanal hervorruft. Um diesem Effekt zu berücksichtigen (zu kompensieren = Gegenkopplung oder ggf. absichtlich zu verstärken = Mitkopplung), wird der andere Kanal in sanfter und geeigneter Weise so gesteuert, dass es zu einer verbesserten Sicht für das andere Auge kommt, aber ohne dass es zu einer erneuten kontralateralen Übertragung auf den ursprünglich beeinflussten Kanal kommt. Dazu liegt eine Dämpfung vor, um ein Aufschaukeln des Systems aus beiden Pupillen und beiden softwaregesteuerten Kanälen zu verhindern. Hierbei werden die Außenlichtsituation, die Arbeitspunkte der beiden Regler, die Transienten/Beleuchtungsänderungen auf den jeweiligen Kanälen (z.B. heller Tag, bewölkter Tag, Nähe zum Regleranschlag) und die Differenz zwischen den Reglern berücksichtigt.

3) Medizinische und psycho-pathologische Indikationen:

a) Für Patienten mit einem relativen afferenten Pupillendefekt (RAPD) kann in der Software des Mikrocontrollers das rechts-links Pupillenverhaltensmuster des Patienten hinterlegt werden, so dass beim Betrieb innerhalb der beiden oben genannten Modi (1 und 2) deren Pupillenverhalten mit der jeweils korrekten LC-Transparenz derart berücksichtig wird, dass die wahrgenommenen Helligkeit immer konstant ist oder bestimmten gewünschten Sollwerten entspricht.

b) Für Patienten mit einem ärztlich verordneten rechts-links Sehtraining (z.B. nach einem Schlaganfall), kann optional eine Seite permanent oder nach bestimmten zeitlichen Mustern wechselweise dunkler oder heller geschaltet werden.

c) Für Einsatzkräfte in Stresssituationen (z.B. Soldaten im Einsatz), die einen akut erhöhten Adrenalinpegel und somit generell geweitete Pupillen haben, kann die Software per Befehl (Taster) die Transmission entsprechend verringern (leicht abdunkeln), damit die visuelle Wahrnehmung bei Helligkeit angenehmer ist.

Die innenliegenden Fotozellen sind mindestens doppelt oder gar dreifach ausgelegt. Dies dient nicht nur zur Berechnung der mittleren Helligkeit im wahrscheinlichsten Ortsmittel der Pupille (wie oben beschrieben), sondern auch aus Sicherheitsgründen. So kann die Software durch logischen Vergleich (z.B. zwei Sensoren zeigen ähnliche Helligkeit und nur einer zeigt gar keine Helligkeit) ggf. eine Verschmutzung oder einen Defekt eines bestimmten Fotosensors erkennen und als Konsequenz nur noch die beiden funktionieren Fotosensoren berücksichtigen.

Dazu enthält die Software zusätzlich zu den permanent berechnenden Regler-Komponenten auch rein logische Sicherheitsroutinen (separate State-Machines), die ständig parallel zum Regeln die Funktion der Brille sicherstellen. (In diesem Zusammenhang sei noch angemerkt, dass die fehlerunanfälligsten Brillen dieser Art, die für automotive Anwendungen gedacht sind, entsprechend der ASIL-Norm zugelassene Dual- oder Tri-Core Prozessoren beinhalten, die sowohl Hardware als auch Software auf Fehler prüfen.

Eye Tracker einfacher Art

In Analogie zu den oben genannten Fotosensoren oder Kameratypen, die ein menschliches Auge simulieren, wird im inneren der Brille, wo sich dieser Sensor befindet, ein zweiter Sensor platziert, der das Auge beobachtet. Dieser könnte z.B. auf der Rückseite des vorgenannten Sensors oder auch leicht versetzt daneben montiert sein. Es können diverse Arten von Sensoren eingesetzt werden, z.B. relativ einfache und kostengünstige Fotosensoren, oder Facettensensoren, oder höher auflösende abbildende Kamerasysteme. Im einfachsten Fall wird die Blickrichtung nur grob erfasst. Insbesondere die links-rechts-Bewegung des Auges lässt sich sogar am weißen Teil des Auges (Lederhaut) einfach detektieren, indem eine codierte Infrarot-Lichtschranke eingesetzt wird. Infrarotlicht wird nicht vom Auge wahrgenommen, aber je nach Blickrichtung unterschiedlich reflektiert. Eine Codierung der IR-Quelle ist notwendig, damit es nicht zur Verwechslung mit anderen Lichtquellen und Reflexionen auf der Empfängerseite kommt. Diese Codierung kann im einfachsten Fall zyklisch sein (z.B. 10 kHz Rechteck mit bekannter Frequenz und Phasenlage). Aus der Frequenz und insbesondere der Phasenlage kann ein Phase-Sensitive Detektor (PSD, auch Boxcar-Amplifier genannt) nach Tiefpass-Integration über ca. 10 Zyklen, also mit immerhin ca. 1 kHz eine sehr genaue Amplitudenmessung bezüglich des Sendersignals machen, selbst dann wenn dieses gegenüber dem "Rauschen" anderer IR Signale sehr schwach ist.

Dies ist nur ein Beispiel für einen einfachen Eye Tracker. Man kann auch die Pupillenstellung mit einem sehr ähnlichen Verfahren ermitteln - ebenfalls in Reflexion, aber dann bezüglich der Absorption in der dunklen Pupille anstelle der Reflexion an der weißen Lederhaut. Da Reflexions-Lichtschranken sehr kostengünstig sind, können durchaus sowohl innen am Auge (Nasennähe) als auch außen am Auge (Schläfennähe) solche Sensoren angebracht werden, ggf. auch noch mittig unter dem Auge (Blick nach oben/unten erfassend) - also durchaus 2 bis 3 Sensoren. Mehrere solcher Sensoren erhöhen die Messgenauigkeit bezüglich der Blickrichtung.

Idealerweise wird jedoch ein Eye Tracker verwendet, der eine winzige hochauflösende abbildende Kamera verwendet, ähnlich wie sie in Smartphones oder Notebooks verwendet werden. Diese Kamera erfasst die Pupillenstellung bezüglich Blickrichtung und somit aller Winkel.

Korrelationsberechnung aus Fotosensoren und Eye Tracker

Die Richtungs- und Helligkeitsinformationen der Fotozellen/Kamera werden mit der durch den Eye Tracker bestimmten Blickrichtung per Software mathematisch korreliert. Dies bedeutet beispielsweise, dass zunächst die Blickrichtung als Ausgangsgröße genommen wird, während zeitgleich (d.h. in Echtzeit) die einfallende Helligkeit unter dem exakt gleichen Winkel gemessen und konstant geregelt wird. Da es sich um einen Echtzeit-PID-Regelkreis handelt, bei dem die Regelabweichung stets Null ist, wird die Helligkeit in Blickrichtung stets konstant sein - nämlich dem eingestellten Sollwert entsprechend.

Wenn diese Regelung sehr exakt funktioniert, was unter Einsatz von Hochtechnologie möglich wäre, würde die Pupille auf der Hauptachse niemals einen Helligkeitsunterschied erfahren. Dieser Regelmodus kann je nach Anwendung (z.B. Sport, Automotive, Industrie, Medizin, Militär) gewählt werden, z.B. durch einen Schalter oder sonstigen Befehl (z.B. über ein Smartphone, welches über Bluetooth o.ä. mit der Brille verbunden ist).

Andererseits könnte dieser extrem schnelle und präzise Regelmodus je nach Anwendung auch zu unerwünschten Artefakten in der Wahrnehmung führen. Daher ist ein alternativer Modus einstellbar, in dem die Software absichtlich etwas verlangsamt wird bzw. die Helligkeit nur in leichten Winkelabstufungen nachgeregelt wird. Beispielsweise würde erst dann, wenn der Anwender wirklich genau in eine eher seitlich liegende Blendquelle (z.B. Autogegenverkehr) schaut, sofort auf konstante Helligkeit geregelt, ansonsten, wenn sich die Pupille nur in geringem Maße in der Mitte hin und her bewegt und aus der Region kein Gegen-Blendlicht rührt, wird nur auf diese Helligkeit konstant geregelt.

Außerdem wird die individuelle und altersabhängige Blendempfindlichkeitsfunktion, welche in der Software als Formel oder Look-Up-Table (LUT) hinterlegt sein kann, wie eine Schablone (z.B. mit multiplikativer Gewichtung) über das Signal des nach vorne schauenden Helligkeitssensors gelegt. Obwohl sich dieser Sensor nicht wie ein Augapfel bewegt, sondern starr geradeaus montiert ist, wird aufgrund des Eye-Tracker-Signals diese Schablone gemäß der Augapfel-Bewegung mitverschoben. Damit ist praktisch ein künstliches Auge geschaffen, welches die individuelle blickwinkelabhängige Blendempfindlichkeit berücksichtigt, welche dem Echtzeit-PID-Regelkreis als Führungsgröße (inwendig ebenfalls "Istwert" genannt) dient. Dabei bleibt es dem Fachmann überlassen, die Algorithmen je nach vorgesehener Anwendung sanfter oder stärker auszuprägen. Alternativ kann vorgesehen sein, dass der Anwender dazu eine Auswahl trifft.

Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente. Im Einzelnen zeigt:

Fig. 1      eine schematische Darstellung in einer Schnitt-Aufsicht der elektronischen Brille;

Fig. 2      ein Diagramm der sog. Transmission der Brille eines Blendunterdrückungssystems über die Zeit, wobei das System mit einer Blendwaffe ausgerüstet ist (Die Blendwaffe ist nicht erfindungsgemäß, sondern dient lediglich der Veranschaulichung);

Fig. 3      eine schematische Darstellung der Situation, wenn ein Blendsignal (Sonne) auf einer Anzeige bzw. Bildschirmoberfläche reflektiert wird (nicht erfindungsgemäß, dient lediglich der Veranschaulichung);

Fig. 4      die Situation aus Fig. 3, mit einer Zusatzvorrichtung für nicht modulierbare Displays (nicht erfindungsgemäß, dient lediglich der Veranschaulichung);

Fig. 5      eine schematische Darstellung der Situation mit einem sog. "innenliegenden HUD" (nicht erfindungsgemäß, dient lediglich der Veranschaulichung);

Fig. 5B      eine Ausführung als Schutzbrille für völlige Dunkelheit, ohne Eigenlichtquelle (Arbeitsschutz);

Fig. 6      ein Diagramm der Transmission für ein Blendunterdrückungssystem mit RGB-Farbcodierung (nicht erfindungsgemäß, dient lediglich der Veranschaulichung);

Fig. 7      ein Diagramm, welches das Verhalten der unterschiedlichen Transmissionsgrade TR (Ch#1, 2, 3) bei einem Blendunterdrückungssystem mit RGB-Farbcodierung (nicht erfindungsgemäß, dient lediglich der Veranschaulichung) verdeutlicht;

Fig. 8      eine schematische Darstellung eines Systems zum Verstärken des räumlichen Eindrucks (nicht erfindungsgemäß, dient lediglich der Veranschaulichung);

Fig. 9      eine schematische Darstellung eines Systems zum Verbessern der Sehweite durch Ausblendung von Reflexionen im Nahbereich bei Partikelniederschlag nach dem LIDAR-Prinzip (nicht erfindungsgemäß, dient lediglich der Veranschaulichung);

Fig. 10      ein Diagramm zur Eigenlichtunterdrückung eines Blendunterdrückungssystems (nicht erfindungsgemäß, dient lediglich der Veranschaulichung); und

Fig. 11      ein weiteres Diagramm zur Eigenlichtunterdrückung, das die Initialisierungsphase zeigt (nicht erfindungsgemäß, dient lediglich der Veranschaulichung).

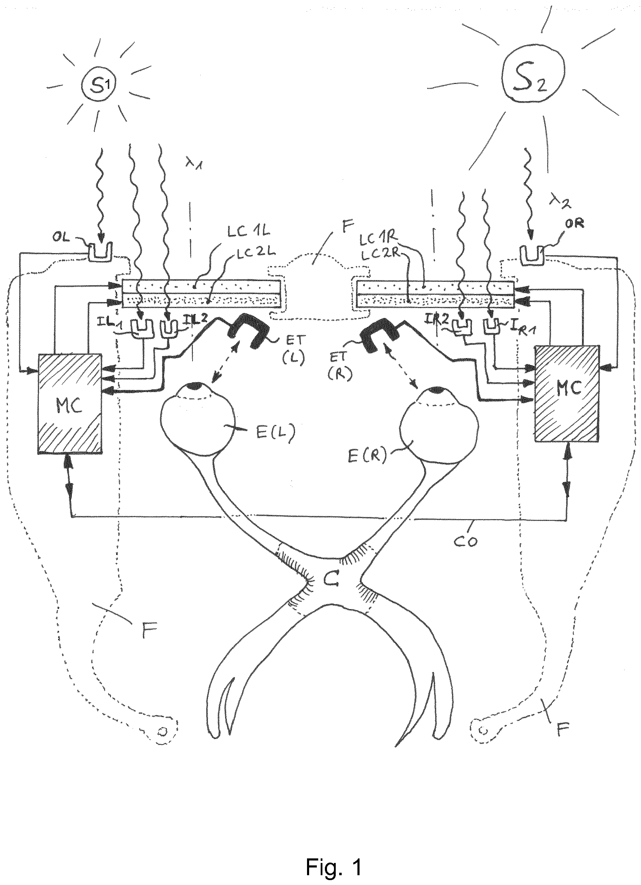

Im Folgenden wird teilweise auf Fig. 1 Bezug genommen.

Alles Nachfolgende gilt immer für ein Auge (rechts oder links, auch als "Kanal" bezeichnet). Ein Kanal besteht aus mindestens einer LC-Zelle (es können aber auch zwei oder mehrere LC-Zellen hintereinander geschaltet werden), welche je nach Anwendung geeignetes schnelles und kontrastreiches LC-Material (TN, STN, Fe-LC) beinhaltet.

Die vom menschlichen Körper entferntere Zelle wird als "distal" bezeichnet, die am Auge befindliche als "proximal". Hinter der proximalen Zelle befinden sich in einem gewissen Abstand (typ. 1-3 mm) ein bis drei komplexe Fotosensoren IL1, IR1, die in Blickrichtung den Lichteinfall durch die LC-Zelle LC 1L, LC 2L, LC 1R, LC 2R aufnehmen, wobei ein einzelner Fotosensor wiederum aus mindestens 3 Sensoren besteht, die ein orthogonales x-y-z-Koordinatensystem aufspannen - wobei der Vektor (1,1,1) sinnvollerweise in Blickrichtung zeigt.

Alternativ zu einem solchen x-y-z-Fotosensor ist es möglich, ein Fotosensor-Array zu verwenden, welches - ähnlich einem Facettenauge - über deutlich mehr als nur 3 orthogonale Kanäle verfügt. Jeder Kanal kann die Helligkeit in einem großen Dynamikbereich messen, so dass dem Mikroprozessor ein "grobes Bild" übermittelt wird.

Alternativ zu einem solchen "groben Bild" kann auch ein mit optischen Linsen abbildendes System (Kamera) mit deutlich höherer Auflösung vorgesehen sein (z.B. 5 Megapixel Kamera) bei gleicher Miniaturbaugröße, die wenige Quadratmillimeter nicht überschreitet, ähnlich wie sie bereits in Smartphones und Notebooks eingesetzt werden. Das von solch einer Kamera an den Prozessor übermittelte Bild ist feiner aufgelöst: Der Dynamikumfang und die Linearität zur Messung der Helligkeit wird durch den Einsatz hochdynamischer Chipmaterialien sichergestellt, ähnlich wie in der analytischen medizinischen Fotographie.

Aus reinen Sicherheitsgründen werden pro Auge E(L), E(R) (Kanal) mindestens 3 solcher komplexen Fotosensoren verwendet (x-y-z, oder Facetten oder Kamera).

Alle genannten Fotosensoren können z.B. als Fotodioden, Fototransistoren, Fotozellen, usw. ausgeführt sein - jedenfalls ist allen gemeinsam, dass sie farbneutral reagieren, indem sie die Farbempfindlichkeitskurve des Auges (sog. V-Lambda-Funktion nach DIN 5031) mit einrechnen. Derartige Fotozellen werden beispielsweise in der Fotographie zur farbneutralen Belichtungsmessung eingesetzt. Per Software kann, je nach Umgebungshelligkeit (gemessen durch einen Außensensor OL, OR, oder abgeleitet aus Stellgröße und Sollwert des Reglers MC) bei hauptsächlicher Dunkelheit ein Look-Up-Table (LUT) in den Rechenalgorithmus einbezogen werden, der die V' Werte für Nachtsicht beinhaltet, so dass der sog. Purkinje-Effekt (erhöhte Blauempfindlichkeit bei Nacht) berücksichtigt wird. Ferner kann die individuelle, altersabhängige Blendempfindlichkeit berücksichtigt werden - dazu gibt es empirische Studien, insbesondere winkelabhängig und altersabhängig (z.B. Adrian and Bhanji 1991 - Illumination Engineering Society of North America).

Freiform-Linse/Kanal oder Software mit Kamera

Die physikalische Umsetzung der o.g. Augenempfindlichkeitsformel kann durch eine entsprechende Freiform-Linse aus transparentem Material (z.B. Glas, Kunststoff, Flüssigkeit, etc.) erfolgen, die derart vor einem Fotosensor angebracht ist, dass er wie das menschliche Auge zur richtungsempfindlichen Messung von Helligkeit eingesetzt werden kann. Es wird also ein "künstliches Auge" kreiert, welches sich ebenso blendungsempfindlich über den Einfallswinkel zeigt wie ein menschliches Auge. Hierzu müssen zwei Faktoren berücksichtigt werden: 1. die V-Lambda- und V'-Lambda-Funktionen (Purkinje-Effekt bei Nacht); 2. die winkelabhängige Blendungsempfindlichkeit.

Anstelle dieser Linse kann vereinfachend auch ein entsprechend per Freiformberechnung geformter schwarzer Kanal (d.h. im Wesentlichen eine Bohrung) verwendet werden, an dessen Ende sich die Fotozelle befindet, so dass diese einen Öffnungswinkel erhält, der der Empfindlichkeit des menschlichen Auges entspricht.

Alternativ kann die Formel zur Blendungsempfindlichkeit rein als Algorithmus bzw. in die Software implementiert werden, die auch das hochauflösende/hochdynamische Bild der Kamera empfängt, da in dem Kamerabild auch die Richtungsinformation und Helligkeit pro Pixel enthalten ist. Das Kamerabild kann dann mit individuellen (altersabhängigen) Bewertungsformeln gewichtet werden, zumal man sein persönliches Alter oder sonstige individuelle Präferenzen oder ärztliche Indikationen/Empfehlungen bezüglich Blendungsempfindlichkeit über eine beliebige Mensch-Maschine-Schnittstelle (z.B. Tasten an der Brille, USB-PC-Software-Schnittstelle, Smartphone-App via (Bluetooth)-Funk) eingeben kann.

Eye Tracker

Die Richtungs- und Helligkeitsinformationen der Fotozellen/Kamera werden zusätzlich mit der Blickrichtung, die durch einen Eye Tracker ET(L), ET(R) bestimmt wird, mathematisch korreliert.

Die individuelle und altersabhängige Blendempfindlichkeitsfunktion, welche in der Software als Formel oder Look-Up-Table (LUT) hinterlegt sein kann, kann dann wie eine Schablone (z.B. mit multiplikativer Gewichtung) über das Signal des nach vorne schauenden Helligkeitssensors gelegt werden. Dieser Sensor ist starr an der Brille montiert. Aufgrund des Eye-Tracker-Signals wird diese Schablone jedoch gemäß der Augapfel-Bewegung mitverschoben, wodurch die Funktionalität eines künstlichen Auges erreicht wird, welches die individuelle blickwinkelabhängige Blendempfindlichkeit berücksichtigt,

Puls-Shaping bei den LC-Zellen

Hier gibt es drei Möglichkeiten:

1. Bei beiden LC-Zellen handelt es sich um Zellen, die im spannungslosen Zustand jeweils transparent sind, um im Falle eines System- oder Spannungsausfalls in jedem Falle normale Sicht zu ermöglichen.

2. Für Hochsicherheitsanwendungen, bei denen von einer permanenten Blendungsgefahr im Arbeitsbereich auszugehen ist (z.B. LASER-Labor oder Lichtbogenschweißen) können LC-Zellen eingesetzt werden, die genau umgekehrt arbeiten, d.h. im spannungslosen Zustand komplett dunkel geschaltet sind und nur durch Drücken eines Sicherheitsschalters oder dergleichen transparent geschaltet werden können.

3. Mischung von Zellen der o.g. Typen, also eine im spannungslosen Zustand durchlässige und eine undurchlässige Zelle. Diese Anordnung kann dazu genutzt werden, die Flankensteilheit bei sowohl der aufsteigenden als auch der fallenden Flanke eines optischen Pulses zu verbessern, im Sinne von Transparentschaltung für einen Bruchteil einer Sekunde in der Form eines Rechteckpulses auf der Zeitachse (Rechteck mit hoher Flankensteilheit auf dem optisch messenden Oszilloskop-Bild). Der Vorteil besteht in geringerem Übersprechen und sonstigen kontrastvermindernden Artefakten (Crosstalk) in Synchron-Anwendungen mit einer Eigenlichtquelle oder mehreren Teilnehmern.

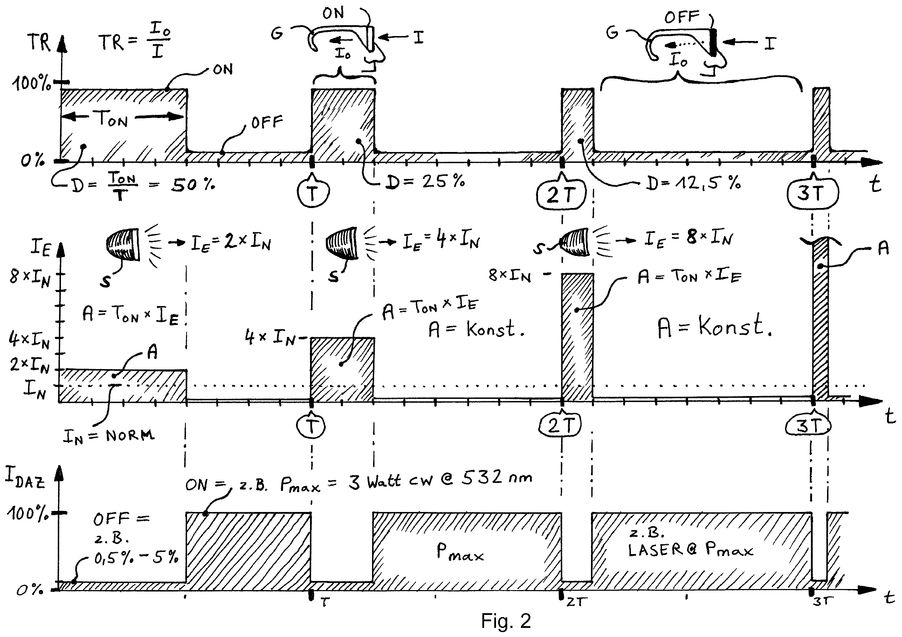

Eine wie vorstehend beschriebene Brille kann als Teil eines Blendunterdrückungssystems Verwendung finden. Fig. 2 zeigt die sogenannte Transmission (TR) einer solchen Brille über die Zeit. Die Transmission ist dabei der Quotient aus der durch die Flüssigkristallzelle LC gelassenen Intensität $I_o$ und der einfallenden Intensität I.

In der Zeit $T_{on}$ ist die Brille geöffnet, d.h. transparent geschaltet. In der restlichen Zeit (Periodenzeit T minus $T_{on}$) ist die Brille geschlossen, d.h. nicht transparent.

Um nahtlose und analoge Grauwerte zu erhalten, wird das Signal in Fig. 2 (erste Zeile) als analoge Pulsweiten-Modulation PWM realisiert, d.h. in Fig. 2 sind von Zyklus T zu den Zyklen 2T und 3T lediglich beispielhaft verschiedene sprunghafte Zustände der PWM abgebildet. Diese Zustände lassen sich auch als prozentuales Puls-Zykluszeit-Verhältnis D (Duty-Cycle) beschreiben.

Um das "Rauschverhältnis" SNR ("Signal to Noise Ratio") zu verbessern, wird die Pulsenergie pro ausgesendeten Lichtpuls in gewissen Grenzen konstant gehalten. Insbesondere soll die Fläche A in der mittleren Zeile von Fig. 2, die sich ergibt aus der aktiven Pulsweitenzeit $T_{on}$ multipliziert mit der jeweiligen emittierten Lichtintensität IE (I = Intensität, E=Emittiert) eines Pulses, weitgehend konstant gehalten werden.

In der Praxis kann dies durch Anlegen einer höheren Spannung bzw. durch Einprägen eines höheren Stroms in ein geeignetes Leuchtmittel, welches für solch hohe Energien ausgelegt ist, erfolgen. Es bleibt dem Fachmann überlassen, sicherzustellen, dass das vorhandene Leuchtmittel hierfür geeignet ist.

Zudem muss die Lichtintensität IE stets dem normierten und bereits von Behörden (TÜV etc.) zugelassenen Intensitätswert I Norm entsprechen, allerdings multipliziert mit dem Kehrwert des Hundertstel des Duty-Cycle D.

Beispiel:

$$\text{Puls-Pause-Verhältnis} = \text{Duty-Cycle} = 50\% = 0{,}5$$

$$\text{Kehrwert von } 0{,}5 = \text{Faktor } 2$$

$$\text{IE} = 2 \times \text{I Norm}$$

Dieses Verfahren ist notwendig, damit die über ein langes Zeitintegral gemessene Intensität im zeitlichen Mittel immer einer konstanten I Norm entspricht. Selbst wenn das zeitliche Messintervall bei den Behörden nur 1 Sekunde betrüge, so wären bei einem 70 Hz Scheinwerfer bereits so viele unterschiedliche Pulshöhen bzw. Puls-Zyklen zeitlich gemittelt worden, dass sich immer der geforderte konstante Lichtwert I Norm ergibt. Integriert man das Signal IE in der mittleren Zeile von Fig. 2 von t = 0 bis zum Zyklus-Ende T3, so wird das Prinzip deutlich.

Darüber hinaus wird bei sehr schmalen Zeitschlitzen, in denen die Brille offen und transparent ist (z.B. 5%), bei entsprechend dunkel eingestelltem Sollwert des Regelkreises, d.h. bei konstant und weit geöffneter Augenpupille, das Auge so lichtempfindlich, dass bereits kleine Leistungen von IE (d.h. IE dividiert durch $T_{on}$) ausreichen, um eine sichtbare Verbesserung der betrachteten Szenerie zu erreichen, während ca. 100 - 5% = 95% des störenden Fremdlichtes unterdrückt werden.

Die im Folgenden genannten Aspekte und Ausführungsformen sind nicht erfindungsgemäß, sondern dienen lediglich der Veranschaulichung.

Das vorliegende Blendunterdrückungssystem kann mit einer Blendwaffe (Dazzler) kombiniert werden. Die unterste Zeile von Fig. 2 bezieht sich auf diese Situation und zeigt, wie der Dazzler ein bezüglich der Öffnungsdauer $T_{on}$ der Brille antizyklisches bzw. invertiertes Einschalt-Signal erhält. Zudem ist zu erkennen, dass der Dazzler auf einen (frei einstellbaren) von Null verschiedenen OFF-Wert in Höhe von z.B. 0,5-5% seiner maximalen Intensität I DAZ festgelegt werden kann, damit er für den Anwender visuell gut verfolgbar bleibt.

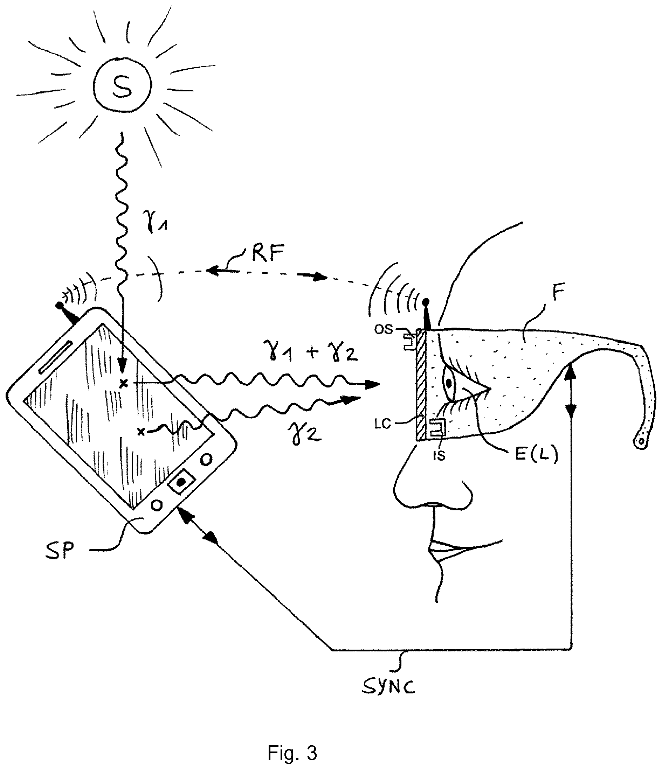

Fig. 3 zeigt, wie das Anti-Blend-System mit einer Anzeige kombiniert werden kann, um Blendungen durch Reflexion an der Anzeige zu unterdrücken und gleichzeitig die Ablesbarkeit der Anzeige sicherzustellen. Hierbei ist das Summensignal Gamma 1 + 2 am Auge stets aus Störsignal und Nutzsignal zusammengesetzt. Im einfachsten Fall kann bereits eine aus dem Internet herunter geladene Software (z.B. eine sog. App) die Display-Hintergrundbeleuchtung eines Smartphones SP oder ähnlichen Geräts, wie z.B. Tablet oder Notebook oder ein außerhalb der Brille befindliches Head-Up-Display, in ihrer Helligkeit derart modulieren, dass das oben beschriebe Anti-Blendsystem realisiert wird. Über 95% des Sonnenlichtes S und Gamma 1 können so unterdrückt werden, während die Lichtpulse des Bildschirms genau in den offenen Zeitschlitz der Brille und auf das dunkeladaptierte Auge fallen.

Die Synchronisierung der Brille mit der Anzeige kann auf verschiedene Weisen geschehen:

1) In einem Falle ist das Elektronik-Gerät der "Master", der einfach gepulstes Licht aussendet, worauf sich die Brille mit Hilfe ihrer Lichtsensoren (Außen = OS, Innen = IS) rein optisch synchronisieren kann.

2) Optional kann über eine Funkverbindung RF zwischen Brille und Endgerät eine Synchroninformation ausgetauscht werden. Typischerweise werden dabei bereits serienmäßig vorhandene Funksysteme, wie z.B. Bluetooth, genutzt. Welches Gerät "Master" ist, kann hier offen bleiben und ist nur eine Frage der Programmierung.

3) Im Übrigen kann auch mit Hilfe eines Kabels (z.B. USB) oder auch auf jede andere erdenkliche Art eine Synchroninformation SYNC zwischen Endgerät und Brille übermittelt werden. Wer von beiden "Master" ist, kann auch hier offen bleiben und ist nur eine Frage der Programmierung.

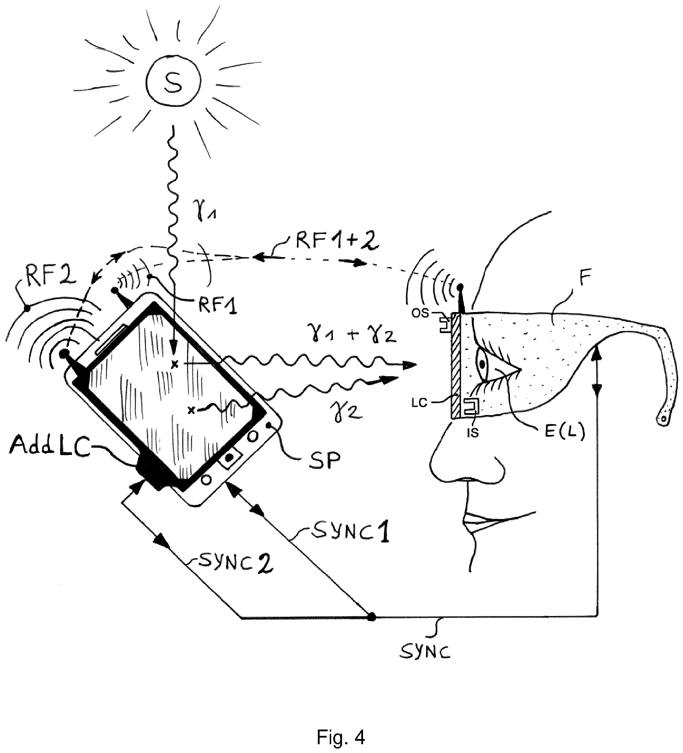

Im Folgenden wird auf Fig. 4 Bezug genommen.

Auch für Displays und Anzeigen, die eine Modulation der Hintergrundbeleuchtung nicht ohne weiteres zulassen, ist eine Lösung möglich. Für Anzeigen, die zumindest eine gleichförmige Hintergrundbeleuchtung besitzen (z.B. papierähnliche Displays mit "elektronischer Tinte" zum Lesen von Büchern), kann ein weiterer Flüssigkristall-Shutter AddLC auf dieses Display aufgelegt oder geklemmt werden. Dieser zusätzliche Shutter moduliert dann das ansonsten gleichmäßige (DC), aber maximale (oder per Eingriff auch übermaximale) Hintergrundlicht des Displays entsprechend der Zeitschlitze der Brille. Sofern die gleichmäßige Hintergrundbeleuchtung sehr hell eingestellt werden kann, ergeben sich mit dieser Anordnung die bereits oben beschriebenen Vorteile der Blendungsunterdrückung von fremden Störlichtquellen S, einschließlich der beschriebenen Verbesserung der Lesbarkeit. Der zusätzliche Shutter verfügt über eigene Schnittstellen zur Synchronisation mit der Brille, z.B. Funk RF2 oder einen Kabelanschluss (z.B. USB) oder einen beliebigen sonstigen Zugang SYNC2.

Im Übrigen kann auch eine geeignete Kombination aus den genannten Informationskanälen zum Einsatz kommen, z.B. eine Software ("App") zum Einschalten der Hintergrundbeleuchtung über Funk RF1, und zur Synchronisation mit der Brille die Funkverbindung RF2 oder das Kabel SYNC2. Auch eine rein optische Synchronisation durch die optischen Sensoren OS, IS der Brille ist möglich.

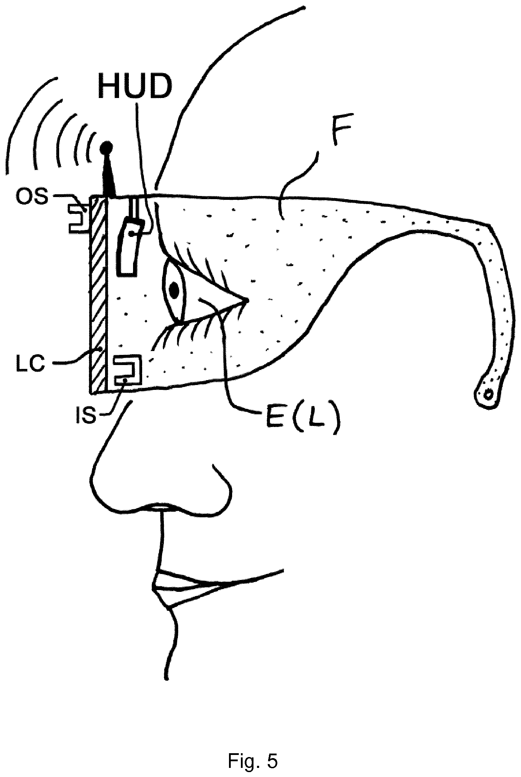

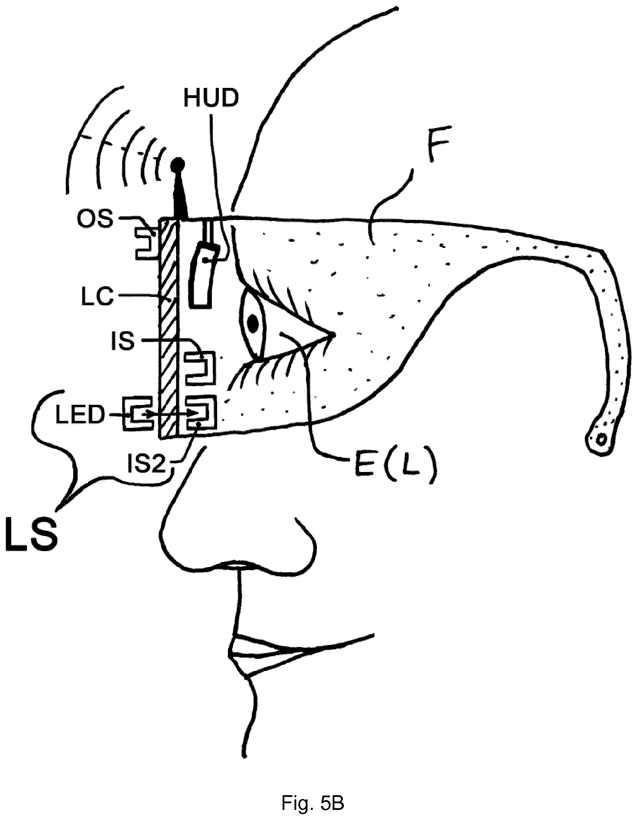

Im Gegensatz zum außerhalb der Brille liegenden Head-Up-Display (HUD) stellt das "innerhalb der Brille liegende HUD" einen Sonderfall dar, der in Fig. 5 dargestellt ist (Durchsicht-HUD, ähnlich wie bei "Google Glass" oder Samsung "Gear Glass" etc.). Hier ergibt sich eine Ableseverbesserung durch Blendunterdrückung, was z.B. beim versehentlichen Blick in die Sonne wichtig ist (Shutter wird kurzfristig ganz oder nahezu geschlossen). Zudem ergibt sich eine Verbesserung dadurch, dass die Brille über einen sehr großen Dynamikbereich stets auf exakt die gleiche Helligkeit regelt (weitgehend konstanter Sollwert), wodurch wiederum das innenliegende Durchsicht-HUD stets die optimale Hintergrundhelligkeit und/oder den optimalen Kontrast erhält, egal wie sich außerhalb die Helligkeit ändert. Das innenliegende HUD ist dadurch jederzeit optimal ablesbar.

Das Folgende bezieht sich auf Fig. 5B.

Es gibt im Rahmen des Arbeitsschutzes sehr einfach arbeitenden Blendschutzbrillen, die hauptsächlich im Dunkeln getragen werden, z.B. in Forschungs- und Entwicklungslaboren, in denen es zur Durchführung der Arbeit dunkel sein muss (Licht- und LASER-Experimente, Bio-Tech), von Hautärzten während einer Therapie mit Lichtpulsen hoher Intensität (Intense pulsed light - IPL Therapy) oder dergleichen. Diese Schutzbrillen sind jedoch für die Arbeitsausführung oft ungeeignet, weil sie nur zwei Zustände kennen, nämlich auf und zu, und zudem falsch reagieren, da zu wenige Fotosensoren außen angebracht sind, die die Flüssigkristall-Gläser lediglich ansteuern, aber nicht in Echtzeit regeln (vgl. z.B. DE 10 2014 107 587). Zudem bleibt der Transmissions-Zustand der Gläser (auf oder zu) bei Dunkelheit unbekannt, da weder eine Steuerung noch eine Regelung zuverlässige "Istwerte" liefern kann. Sogar eine Regelung hätte in völliger Dunkelheit (z.B. um Null Lux herum) das Problem, dass der Istwert zu klein sein kann, um zuverlässige und sicherheitsrelevante Aussagen über die korrekte Funktion der Flüssigkristallzellen zu machen.

Für solche Situationen wird pro Augenglas (also links und rechts) eine aktive Lichtschranke LS vorgesehen, bestehend aus einen aktiven Lichtemitter LED und einem gegenüberliegenden, weiteren Innen-Sensor IS2, womit durch die Flüssigkristallzellen hindurch deren Transmission konkret über einen weiten analogen Dynamikbereich gemessen werden kann - selbst bei völliger Dunkelheit.

System zur Blendunterdrückung mit RGB-Codierung

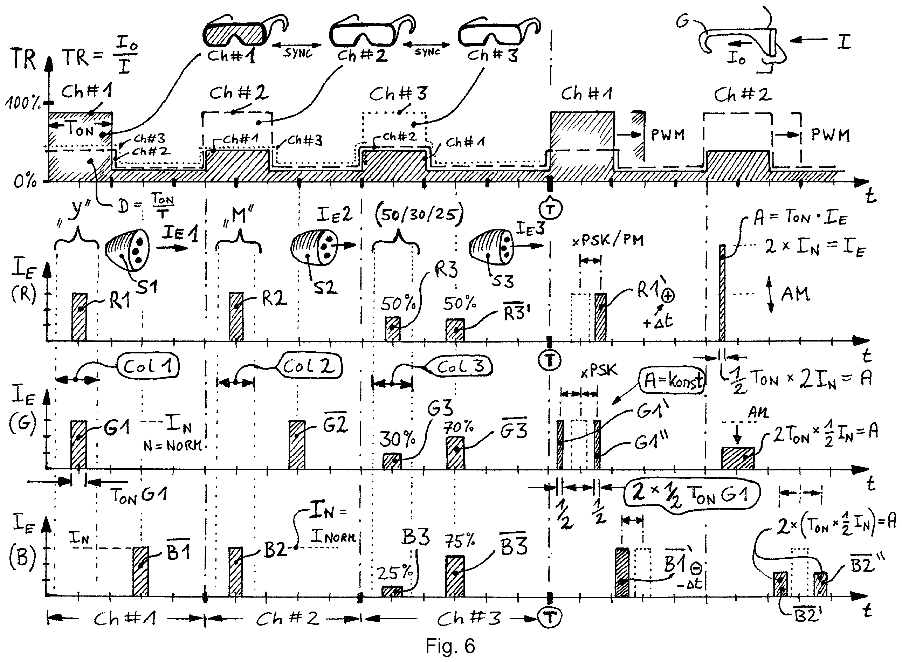

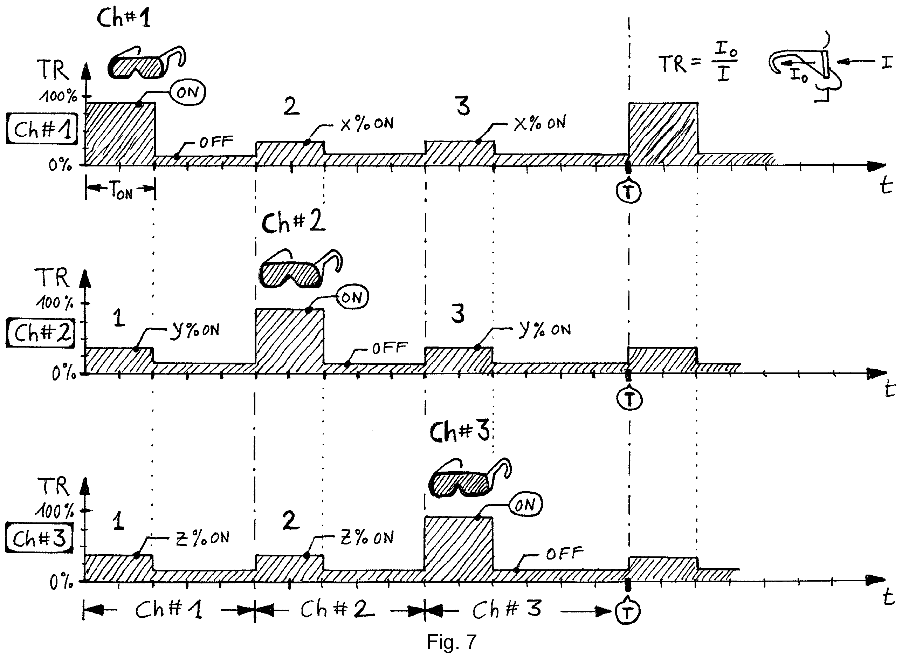

Im Folgenden wird auf die Figuren 6 und 7 Bezug genommen.

Insbesondere bei Blendunterdrückungssystemen, die für Gruppenanwendungen für den Einsatz bei Behörden und Organisationen mit Sicherheitsaufgaben (BOS) oder beim Militär vorgesehen sind, kann eine Ausführungsform zum Einsatz kommen, die ermöglicht, z.B. verschiedenen Teilnehmern oder verschiedenen beleuchteten Objekten (z.B. markierte Ziele) jeweils eine frei wählbare Lichtfarbe zuzuordnen, die beispielsweise nur ein Teammitglied in aller Deutlichkeit zu sehen bekommt - und in abgeschwächter Form auch seine Gruppenmitglieder, während das verwendete Licht für Außenstehende weiß erscheint.

Hierzu werden Eigenlichtquellen verwendet, die nicht nur in ihrer Amplitude bzw. Leuchtintensität moduliert werden können, sondern auch in ihrer Farbe (Wellenlänge). Neben Wellenlängen-durchstimmbaren Lichtquellen wie Oszillatoren (z.B. OPO, OPA-Laser etc.) bieten sich im einfachsten Falle leistungsstarke RGB-LASER oder RGB-LED an, die typischerweise über 3 separat ansteuerbare Kanäle verfügen - nämlich für die sogenannten Primär-Farben "rot, grün und blau", gemäß RGB-Farbmodell, die in entsprechender Überlagerung weißes Licht ergeben. Auch andere Arten und Kombinationen von Primärfarben in der Nähe des RGB-Farbmodells sind denkbar, solange sie in der Summe weißes Licht ergeben.

Die Farben R=Rot, G=Grün, B=Blau des ersten Kanals Ch#1, jeweils getrennt dargestellt in den unteren 3 Diagrammen von Fig. 6 (IE von R, G, B), werden nicht notwendigerweise absolut zeitgleich ausgesendet, sondern es kann beispielsweise Blau auch mit einer leichten zeitlichen Verzögerung nach Rot und Grün ausgesendet werden, jedoch so kurz danach (wenige Millisekunden), dass das menschliche Gehirn dies nicht als Flackern wahrnimmt, sondern stets gemeinsam als weißes Licht.

Der Unterschied für den Träger der Brille mit der Kanalbezeichnung besteht jedoch darin, dass in dem Zeitschlitz $T_{on}$, in dem die Brille geöffnet ist (also TR nahe 100%), die beiden Farben Rot und Grün von der Eigenlichtquelle ausgesendet werden, die Farbe Blau aber erst dann, wenn die Brille bereits wieder geschlossen ist (TR nahe 0% = OFF). In Fig. 6 ist dieser Blau-Puls bezeichnet mit "B1 und Oberstrich", wobei der Strich über dem Buchstaben "negiert" bedeutet. In diesem Kontext heißt B1 negiert "Blau, unsichtbar für Kanal 1". In Fig. 6 ist dies symbolisch mit Y oberhalb der geschweiften Klammer angedeutet, weil die Summe aus Rot und Grün die Mischfarbe Gelb (Engl. Yellow) ergibt. Der Träger der Brille Ch#1 sieht also gelbes Licht. Es wird also wenigstens ein Mehrkanal-Zeitmultiplex-Verfahren bezüglich der 3 Farbkanäle RGB und der jeweiligen Brillen verwendet.

In Fig. 6 ist im Kanal 2 bzw. erkennbar, dass sich hier die Farben Rot und Blau R+B in dem Zeitschlitz $T_{on}$ mischen, in dem die Brille offen ist, angedeutet durch die geschweifte Klammer mit M (für Magenta, da diese Farbe aus der Mischung von Rot und Blau entsteht). Der Träger der Brille Ch#2 sieht also magentafarbenes Licht.

Damit der Träger der eine Idee davon bekommt, welches Ziel sein Nachbar mit Kanal gerade anleuchtet (z.B. zwecks geheimer Markierung), wird die Brille Ch#1 im Zeitschlitz des Kanal nur ein wenig aufgehen, z.B. von nahe 0% (Brille geschlossen) auf (beispielsweise und frei einstellbare) 25% Transmission, so dass auch der Träger die Farbe Magenta des Trägers von Brille Ch#2 sieht. Da aber hiervon nur 25% sichtbar sind, kann sich der Träger von Brille Ch#1 besser auf sein eigenes Licht konzentrieren. Je nach spezieller Anwendung lässt sich der Grad dieser Abschwächung frei verändern zwischen 0% (andere Teammitglieder ausblenden) und 100% (alle anderen genauso hell sehen wie die eigene Farblichtquelle).

Eigentlich überlappen sich die "zeitgleichen Signalflanken" (durchgezogene, gestrichelte und gepunktete Linien in Fig. 6). Zur besseren Erkennbarkeit wurden diese in Fig. 6 jedoch nicht überlappend gezeichnet, sondern minimal versetzt. Die korrekte Situation, ohne diesen Versatz, wird in Fig. 7 verdeutlicht. Dort ist zu erkennen, dass jede Brille bzw. jeder Kanal Ch#1 bis Ch#3 in Wirklichkeit ungefähr gleich breit ist (gleiches $T_{on}$), und dass im Zeitschlitz der anderen Kanäle die jeweilige Brille ganz leicht öffnet (z.B. etwa 25%). Fig. 7 stellt also dieselbe Situation dar wie Fig. 6, allerdings mit separaten Kanälen. Die Variablen x%, y%, z% sollen hierbei darstellen, dass jeder Nutzer den Grad der Erkennbarkeit der anderen Teilnehmer bzw. Farben frei einstellen kann - je nach seiner Rolle im Team oder nach persönlichen Präferenzen.

In Fig. 6 sind nach Ablauf der Zykluszeit T diverse beispielhafte Modulationsverfahren für die RGB-Lichtquellen dargestellt: In Analogie zu der eingangs beschriebenen Methode der konstanten Energie pro Puls (konstante Pulsfläche A) kann auch eine RGB-Lichtquelle so moduliert werden, dass die einzelnen Farbkanäle zeitlich schmaler, aber dafür in der Intensität höher werden und/oder umgekehrt. Dies ist problemlos möglich, weil RGB-LED oder RGB-LASER relativ schnell, insbesondere deutlich hochfrequenter als die Brille, in Phase und Amplitude moduliert werden können. Somit kann die exakte Phase (zeitliche Position) eines einzelnen RGB-Pulses innerhalb der Öffnungszeit $T_{on}$ der Brille pro-blemlos variiert werden, sei es von Gesamt-Zyklus zu Gesamt-Zyklus (ca. 70-140 Hz), oder sogar extrem schnell (>> 1 kHz) innerhalb eines Zyklus. Durch eine solche extrem schnelle Phasenvariation kann man eine Phasenmodulation oder eine PSK auf jeden einzelnen RGB-Kanal aufbringen, die von anderen Brillen oder sonstigen Empfängern erkannt werden und z.B. auch zur "optischen Synchronisation" der Brillen herangezogen werden kann - die Außen- und Innensensoren OS, IS der Brillen sind stets schnell genug dafür. Damit lässt sich eine Synchronisierung der Brillen innerhalb eines Teams auch ohne Funkkontakt herstellen (z.B. falls dieser unerwünscht ist oder ausfällt).

Abgesehen von der Farbmarkierung von Objekten kann diese Phasenmodulation zusätzlich mit einem geheimen Schlüssel und geheimen Informationsinhalten derart codiert sein, dass auch sonstige Informationen (z.B. um welche Art von Objekt es sich handelt, Name, etc.) im Sinne einer vollständigen Markierung ("full information designation") auf ein Ziel oder Objekt aufgebracht werden. Diese vollständige Information kann wiederum von den Außen- und Innensensoren OS, IS oder auch von gesonderten Empfangs- und Dekodier-Einheiten entschlüsselt werden.

In Fig. 6 ist auf dem dritten Zeitstrahl von oben (IE Grün) rechts, jenseits der Periodendauer T die Aufspaltung des Grün-Impulses in zwei zeitlich halb so breite Pulse G1' und G1" zu sehen (d.h. $2 \times \frac{1}{2} T_{on}$), rechts oberhalb bezeichnet mit A = konstant, was dem schon erklärten Prinzip der konstanten Energie pro Puls entspricht. Darüber steht zudem "xPSK", was bedeutet, dass mit zwei getrennten Pulsen, ähnlich wie "Di-Bits", welche in Phasenrelation zueinander oder auch in Relation zur Zeitachse variieren und springen können, nahezu x-beliebige PhasenModulationsverfahren möglich sind - theoretisch auch QPSK und ähnliche Verfahren.

Auf dem unteren Zeitstrahl (IE Blau) ist ganz rechts außen die Aufspaltung des Blau-Pulses in B2' und B2" (jeweils oben negiert) zu sehen, allerdings nur in halber Höhe, d.h. Amplitude 0,5 l Norm. Auch in diesem Beispiel wird deutlich, dass die Fläche A (also die Energie des Pulspaares) konstant bleibt. Die Amplitudeninformation kann, wie bei einer Amplitudenmodulation AM ebenfalls zur Informationsübertragung genutzt werden, ggf. auch mit einem geheimen Schlüssel kodiert. Auch Mischformen aus beliebigen FSK-, x-PSK- und AM-Verfahren sind daher möglich.

Die Synchronisierung der Brillen und Eigenlichtquellen erfolgt in der Regel über Funksignale, kann aber auch optisch erfolgen. Die Synchronisierung kann dabei nach einem bestimmten Hierarchie-System erfolgen, wonach ein Teilnehmer stets "Master" ist und alle anderen stets "Slave" (bei Ausfall des Master könnte nach einem einprogrammierten Rangprinzip ein bestimmter anderer "Slave" zum neuen "Master" werden usw.). Diese Hierarchie kann z.B. im Rahmen einer gemeinsamen Initialisierungsroutine (also zeitlich vor einem Einsatz) festgelegt werden, aber auch mittendrin im Geschehen (z.B. über Funk oder optisch, aufgrund einer einprogrammierten codierten Erkennung, ähnlich wie man es von Multi-User-IT-Systemen wie LAN, WLAN, Token-Ring etc. bereits kennt).

Zudem kann dieses Multi-User-Gesamtsystem auf Kosten einer etwas geringeren Kanalanzahl so betrieben werden, dass der Pulsweiten-Modulationshub der Brille etwas erweitert wird (siehe Fig. 6 oben rechts im Diagramm TR, rechts jenseits der Periode T, gekennzeichnet mit gestrichelter Flanke und PWM. Diese Erweiterung des PWM-Modulationshubes hat den Vorteil, dass die Brille bei leichter Dämmerung (z.B. 0 Lux bis 100 Lux) weiterhin mit analogen Graustufen geregelt werden kann. Selbst bei einer Multikanal-Gruppenanwendung mit unsichtbarer Farbmarkierung kann die Brille zwecks analogen Graustufenregelbetriebs nahtlos in Richtung einer Tagfahrbrille (wie weiter oben beschrieben) betrieben werden.

Die Eigenlichtquelle muss nicht notwendigerweise ausschließlich aus leistungsstarken RGB-LEDs oder RGB-LASERn bestehen, sondern kann auch aus Hochleistungs-Weißlicht-LEDs bestehen, die beispielsweise den Hauptanteil des Eigenlichts ausmachen, während die rot-grün-blau-Komponenten nur additiv zwecks Farbgebung hinzugemischt werden. Dies kann erreicht werden, indem sich im Scheinwerfer/Reflektor neben den Weißlicht-LEDs auch mindestens eine oder mehrere RGB-LEDs/LASER befinden.

In dem kurzen Zeitschlitz $T_{on}$, in dem die eigene Brille offen ist, wird von der Eigenlichtquelle neben dem bereits in Fig. 2 (mittlere Zeile) dargestellten Weißlichtimpuls gleicher Fläche auch eine bestimmte Farbe ausgesendet, d.h. die beiden Modulations-Verfahren (Weißlicht und unsichtbare Farbmarkierung) lassen sich kombinieren, so dass es sich nach wie vor um ein nahtlos funktionierendes Gesamtsystem handelt. Auch eine (weiter oben beschriebene) Blendwaffe kann nach wie vor parallel zu der hier beschriebenen unsichtbaren Farbmarkierung benutzt werden, da diese nur dann eingeschaltet ist, wenn die Brillen sämtlicher Kanäle (Ch#1, 2, 3, etc.) jeweils geschlossen sind (minimale Transmission).

Optional dazu kann die Eigenlichtquelle, wie schon weiter oben beschrieben, nach wie vor mit einem geheimen Puls-Sprungverfahren versehen werden, so dass z.B. gegnerische Einheiten die Farben nicht dekodieren können und auch das Gesamtsystem (Scheinwerfer mit Brille) nicht stören können. Ein solches Gesamtsystem kann selbstverständlich auch mit der verbesserten Ablesbarkeit von Displays kombiniert werden (Figuren 3 bis 5).

Verstärken des räumlichen Eindrucks

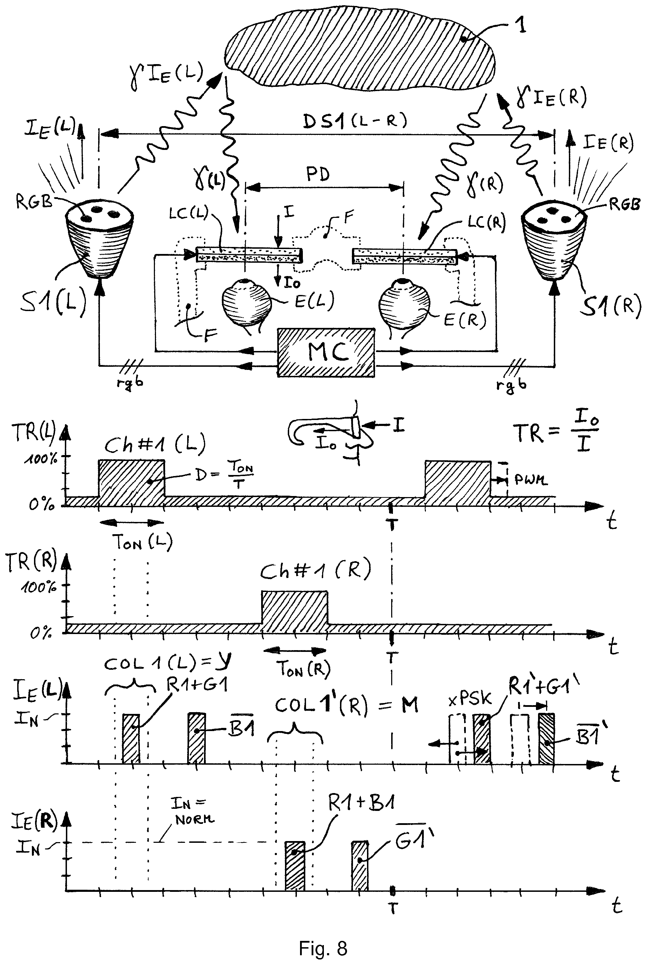

Objekte in großen Entfernungen erscheinen aufgrund des begrenzten menschlichen Augenabstandes zunehmend eindimensional, wodurch ihre Erkennbarkeit eingeschränkt wird. Eine Ausführungsform des erfindungsgemäßen Gesamtsystems, welche hier Abhilfe schaffen kann, ist in Fig. 8 dargestellt. Dort ist der Augenabstand bzw. die Pupillendistanz PD zu sehen, sowie ein beliebiges Objekt 1, welches sich z.B. einige hundert Meter entfernt befindet (auch wenn es sich aufgrund der begrenzten Zeichnungsgröße unmittelbar vor der Brille F zu befinden scheint) - je nach Reichweite der beiden separierten Eigenlichtquellen S1(L) und S1(R). Wie weiter oben beschrieben, kann die Brille F für beide Augen die Helligkeit völlig getrennt voneinander (d.h. zwei voneinander getrennte Kanäle/Regelungen) in Echtzeit regeln, dies sogar unter Berücksichtigung von absichtlichen Helligkeitsdifferenzen (HDR-Sehen) und/oder physiologischen Besonderheiten. Nun ist allerdings vorgesehen, dass der Mikrokontroller MC auch zwei getrennte Eigenlichtquellen ansteuern kann. Diese sind rechts und links vom Träger eines solchen Systems angeordnet, allerdings in einem größeren Abstand DS1(L-R) als der Pupillenabstand PD des Trägers.

Die Arbeitsweise entspricht dabei im Wesentlichen der weiter oben beschriebenen RGB-Codierung. Die Flüssigkristalle der Brille werden dabei wechselseitig nacheinander, aber niemals gleichzeitig geöffnet, so wie im Diagramm TR(L) und TR(R) dargestellt. Da es sich nach wie vor um ein Zeitmultiplex-Verfahren handelt, geht dies auf Kosten der freien Kanäle (Nutzer), so dass das System in etwa nur halb so viele Nutzer in einer Gruppenanwendung verarbeiten kann, sofern alle Teilnehmer die 3D-Verstärkung nutzen wollten. Im Unterschied zur weiter oben beschriebenen RGB-Codierung wird hierbei allerdings pro Auge eine deutlich voneinander unterscheidbare Farbe verwendet, z.B. Gelb Y links und Magenta M rechts.

Aus Platzgründen wurde in Fig. 8 nicht jeder einzelne RGB-Kanal aufgezeichnet, sondern direkt die Farbsumme pro Augenkanal L, R, erkennbar z.B. an der Bezeichnung R1+G1 im linken Kanal IE(L). Im Totzeitschlitz (beide Brillengläser sind geschlossen), folgt der Lichtpuls B1 (negiert), damit für außen stehende Dritte das Gesamtsystem in neutralem Weißlicht erscheint. Auf dem rechten Augenkanal IE(R) addieren sich beispielhaft R1+B1 zu M (Magenta), im Totzeitschlitz (beide Augengläser geschlossen) gefolgt von einem Grünpuls G1 (negiert). Das Grundprinzip ist also prinzipiell identisch mit der RGB-Codierung, auf deren Beschreibung weiter oben zur weiteren Vertiefung verwiesen sei. In Fig. 8 sind außerdem rechts, jenseits der Periodendauer T bereits beschriebenen Phasenmodulationsverfahren und xPSK-Verfahren angedeutet.

Insgesamt führt dieses Verfahren zu einer besseren 3D-Wahrnehmung, die in der Fachliteratur allerdings häufig als "2,5D" bezeichnet wird, da man nicht vollständig hinter das Objekt schauen kann.

Das Verfahren funktioniert auch mit einem Gemisch aus moduliertem Weißlicht und RGB-Licht, so dass das System zum weiter oben genannten Mischen von hochfrequenten RGB-LED/LASER-Modulen mit etwas langsameren Weißlicht-LEDs kompatibel ist.

Die Verwendung von reinem Weißlicht (d.h. ohne RGB-Quellen) ist ebenfalls möglich, insbesondere indem der Abstand der Quellen DS1(L-R) noch mehr vergrößert wird und/oder indem man den linken und rechten Kanal wechselseitig deutlich wahrnehmbar aufblinken oder wechselseitig hin und her blinken lässt (z.B. mit 2 bis 10 Hz), was durch entsprechende Ansteuerung der Eigenscheinwerfer und der Brille möglich ist.

LIDAR

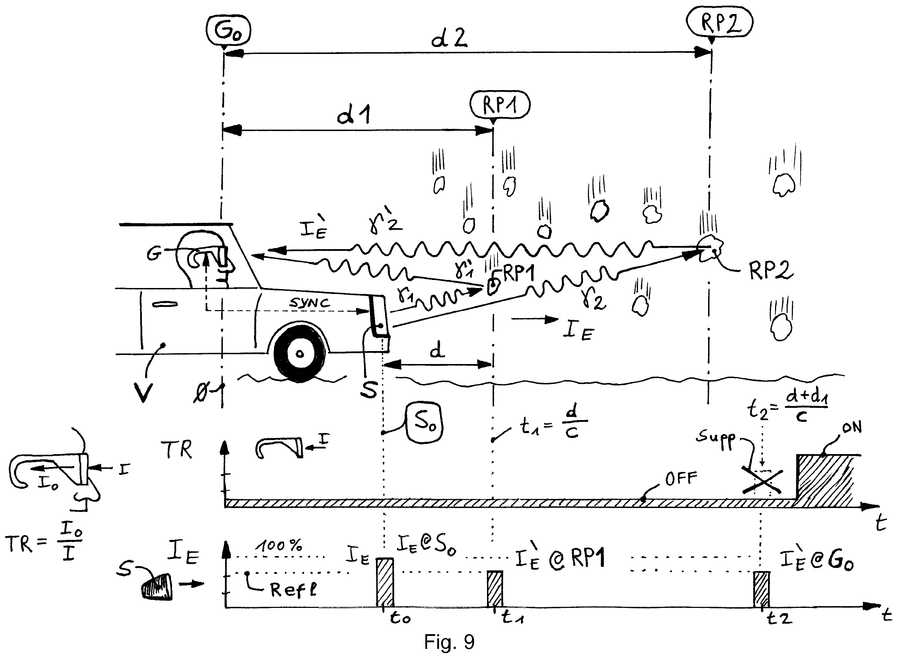

Das bisher beschriebene System kann derart erweitert werden, dass Lichtreflexionen von herunterfallenden oder aufsteigenden Partikeln im Nahbereich des Nutzers ausgeblendet werden. Das Problem tritt beispielsweise beim nächtlichen Autofahren bei Schneetreiben auf, wobei die Schneeflocken unmittelbar vor den Scheinwerfern aufgrund der höheren Leuchtdichte besonders hell erscheinen, ja geradezu blenden, so dass die Sicht auf größere Entfernung, in die Raumtiefe hinein, behindert wird. Diese Situation ist in Fig. 9 dargestellt: In einer Entfernung d1 reflektiert ein Reflexionspartikel RP1 das Licht Gamma1 in Richtung Fahrer.

Werden mit speziellen LASER- oder LED-basierten Scheinwerfern ultrakurze Pulse mit Pulsweiten von wenigen Nanosekunden erzeugt, so können diese nach dem (aus dem Stand der Technik bekannten) LIDAR/LaDAR-Prinzip über ihre Laufzeit mit Hilfe eines ebenso schnellen Shutters für den Nutzer aus- oder eingeblendet werden. Dazu wird die Shutterbrille so angesteuert, dass sie erst zu der (späteren) Zeit t2 öffnet, nachdem die Reflexion des Eigen- bzw. Scheinwerferlichtes an dem räumlich nahen Partikel RP1 zeitlich abgelaufen ist. Die Zeitachse in Fig. 9 ist dabei auch wie eine räumliche Achse zu verstehen, da nach Multiplikation mit der konstanten Lichtgeschwindigkeit c sich die Strecken ergeben (d = ct), und umgekehrt sich die entsprechend Zeiten t ergeben, wenn man die Summe der vom Licht zurückgelegten Strecken durch die konstante Lichtgeschwindigkeit c dividiert (t2 = (d + d1)/c). Nachdem das Licht die Strecken d (Scheinwerfer bis naher Partikel) und d1 (naher Partikel bis Brille) durchlaufen hat, ist also die Zeit t2 verstrichen. Wenn der Shutter der Brille aber erst nach Verstreichen der Zeit t2 öffnet, so wie in Fig. 9 mit TR (= on) dargestellt, so wird der Lichtreflex unterdrückt (supp in Fig. 9) und ist dementsprechend nicht sichtbar.

Schneeflocken oder sonstige Partikel (oder auch Nebel) werden dadurch nicht wirklich unsichtbar - sie erscheinen vielmehr als schwarze Punkte -, aber die Gesamtsicht in die Raumtiefe hinein wird aufgrund verminderter Blendung deutlich verbessert.

Eigenlichterkennung bzw. -Unterdrückung

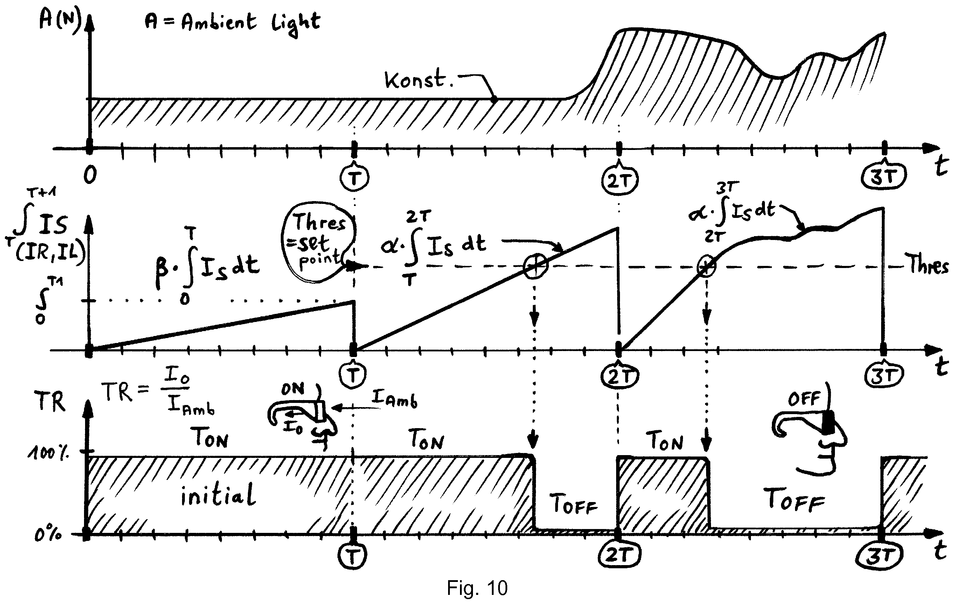

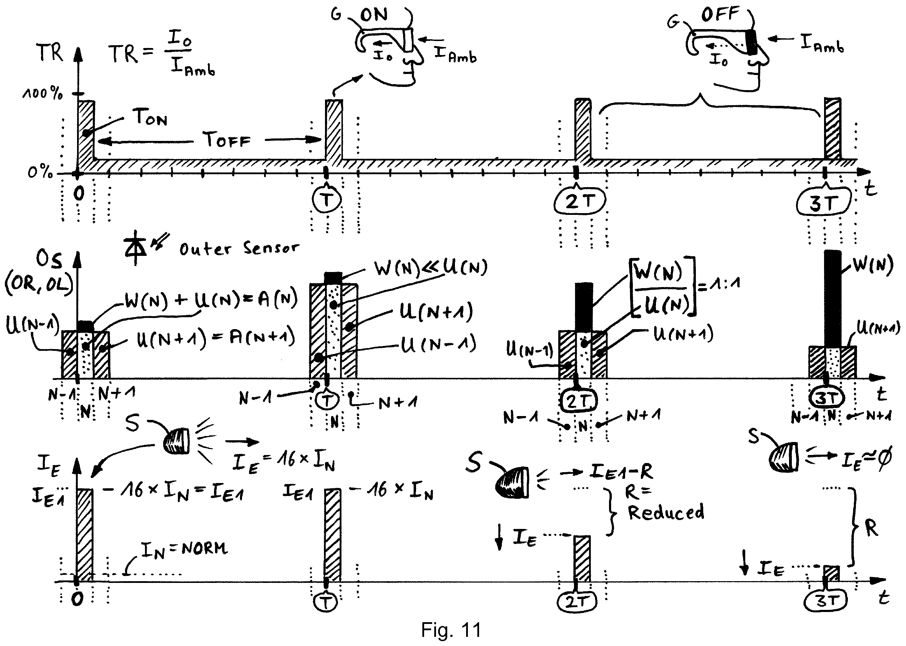

Im Folgenden wird auf die Figuren 10 und 11 Bezug genommen. A bedeutet Umgebungslicht (ambient), U Fremd- oder Störlicht (unwanted, z.B. Sonnenlicht), und W Eigenlicht (wanted). Die Unterscheidung zwischen U und W erfolgt wie nachfolgend beschrieben:

Da der Microkontroller die Zeitpunkte kennt, in denen er den eigenen Scheinwerfer W einschaltet, kann er den äußeren Fotosensor, der mehr als hinreichend schnell ist, in einem Zeitschlitz kurz vor (oder kurz nach) dem ausgesendeten Lichtpuls abfragen - dargestellt in Fig. 10 durch N-1 oder N+1, wobei N den Nten Zeitschlitz des ausgesendeten Lichtpulses beschreibt. Es gilt:

$$A(t) = U(t) + W(t) \qquad (1),$$

beziehungsweise diskret abgefragt, mit N = Mittelwert aus einem Zeitschlitz N gemäß Fig. 10:

$$A (N) = U (N) + W (N)$$

Es wird unterstellt, dass sich das Störlicht zeitlich "kurz vor oder kurz nach" dem Lichtpuls nicht wesentlich ändert, da der Zeitraum zwischen N-1 und N und N+1 sehr klein ist.

$$U (N) = U (N-1) = U (N+1) \qquad (3)$$

Es können auch andere, z.B. komplexere, erfahrungsbasierte Mittelungsverfahren gewählt werden, oder auch das einfache arithmetische Mittel. Jedenfalls wird davon ausgegangen, dass mit dieser Methodik der Störlichtwert Wert U (N) im Zeitschlitz N mit sehr hoher Genauigkeit ermittelt werden kann, sofern das Umgebungslicht sich nicht sehr schnell ändert und nicht seinerseits gepulst ist. Geht man nun davon aus, dass das Zusatzlicht vom Eigenscheinwerfer sich zum Umgebungslicht addiert gemäß Formel (1), dann gilt für A (N), dass diese stets grösser ist als das Umgebungslicht in den Nachbarzeitschlitzen:

$$A (N) > A (N-1) \text{ und } A (N) > A (N+1)$$

Weiterhin gilt, dass die normale Eigenlicht-Rück-Reflexion von entfernten und nicht sehr spiegelnden Gegenständen, d.h. von einer normalen Szenerie / Umwelt (Strasse, Wald, Feld, im Haus mit großen Räumen), eher gering ist, verglichen mit einem massiven Störlicht wie einer tiefstehenden Sonne, so dass im extremen Einsatzfall der massiven Blendunterdrückung gilt:

$$W (N) << U (N) \qquad (6)$$

Oft spricht man bei sehr kleinen Größen auch von einem "Delta", welches hinzukommt oder wegfällt, so dass hier die Formel (1) auch geschrieben werden kann als:

$$\text{Delta } (N) = W (N) = A (N) - U (N) \qquad (7)$$

Da bei einem 70 Hz System binnen einer Sekunde immerhin 70 Mal ein Delta (N) gemessen wird, können diese Werte ihrerseits gemittelt werden, z.B. über einen sinnvollen kleinen Zeitraum, der schnell genug ist, um das Auge hinreichend zu schützen, bezüglich potentiellem Notausschalten oder Herunterregeln der Eigenscheinwerfer beim versehentlichen Blick in diesen Scheinwerfer, z.B. über einen Zeitraum von einem Drittel oder einem Achtel einer Sekunde (x = z.B. 125 ms bis 300ms):
Mittelwert: MDelta (N) = MW (N) = z.B. fließender arithmetischer Mittelwert von allen W (N) in Zeitraum T = t bis t+x

Dieser Wert kann dann einer Schwellwert-Abschaltung zugeführt werden - oder für eine gleichmäßigere (analoge) Herunterregelung der Eigenscheinwerfer herangezogen werden.

Beispiel:

S = Entscheidungsschwelle zur Notausschaltung der Eigenscheinwerfer
W (N) < S Eigenscheinwerfer läuft normal weiter
W (N) >= S Eigenscheinwerfer wird abgeschaltet
Als Faustformel kann gelten, dass ein um ein empirisch ermitteltes Vielfaches M (Multiplikator) heller erscheinendes Licht als Schwelle dient:

$$S = M * U (N) \qquad (8.1)$$

Oder - falls man sich nicht auf U (N) beziehen will, d.h. sich von sogenannten "Szenarien", wie massive oder keine Blendung unabhängig machen will - dann formuliert man einfach selbstbezüglich durch Vielfache von W(N), z.B.:

$$S = 50\% \text{ bis } 500\% \text{ vom üblichen Erfahrungswert von W(N) } (8.2).$$

In Fig. 10 wird angenommen, dass die Brille sich im "Nachtmodus" am Regelanschlag befindet, so dass alle $T_{ON}$ Zeiten gleich schmal sind (z.B. 5% von der Zykluszeit T). Als voll ausgefüllter schwarzer Balken ist in dem Graph in der Bildmitte das erwünschte Licht W(N) dargestellt. Da eine Rückreflektion von einem Objekt in Normalfall nur sehr schwach ist, ist der schwarze Balken für die ersten beiden Zyklen sehr klein dargestellt. Gleichgültig wieviel sonstiges Störlicht U hinzukommt, beispielhaft dargestellt im Zyklus T, das unten dargestellte Scheinwerferlicht bleibt auf konstanter Intensität IE1, d.h. der Scheinwerfer hat mit 16 x IN beispielsweise schon sein Intensitätsmaximum erreicht, welches nicht weiter gesteigert werden kann. Verändert sich jedoch das Verhältnis von Wanted zu Unwanted erheblich, wie in 2T dargestellt (1:1), so wird die Scheinwerferintensität reduziert R. Im Extremfall 3T kann bei direktem Blick in den Eigenscheinwerfer auch abgeschaltet werden (IE nahe Null).

Messung mit dem Innensensor IS - in Verbindung mit kurzen einmaligen Eigenlichtaussetzern

Außerdem kann man alternativ zum obigen Verfahren oder zu Testzwecken in einem Zyklus T wie oben beschrieben das Delta, also W(N), messen, um dann ausnahmsweise und ausschließlich nur in dem Folge-Zyklus 2T die Lampe S anstelle des erwarteten Lichtpulses aus lässt, also einen Lichtpuls als Schlupf bewusst aussetzt. Weil solch ein vereinzelter "Aussetzer" im Zyklus 2T nur ein einziger von insgesamt 70 Lichtpulsen pro Sekunde ist (bei einem 70 Hz System), wird dies vom Nutzer und von außenstehenden Dritten nicht bemerkt.

Liegt DC Störlicht vor oder läuft die Brille synchron zu einem AC Störlicht, kann man sogar voraussetzen, dass sich das Störlicht nicht nur in dem sehr kurzen Zeit-Intervall N-1, N, N+1 kaum ändert, sondern sogar von einem Zyklus T bis zum nächsten Zyklus 2T weitgehend konstant bleibt:

$$U(N, T) = U(N, 2T) \ (9)$$

Der Innen-Sensor IS kann dann im Zyklus T das Delta W(N) messen, während im Zyklus 2T dieses Delta W(N) wegen des ausgeschalteten Eigenscheinwerfers nicht mehr erscheint. Somit kann im gleichen Zeitschlitz N, mittels Innensensor, eine Zusatzmessung von W(N) erfolgen, ohne dass man auf die oben erklärte Messung mit dem Außensensor (in den Zeitschlitzen N-1, N, N+1) angewiesen ist. Wendet man beide Verfahren (also Innensensor mit einmalig ausgeschalteter Lichtquelle und Außensensor mit Messung von A (N-1, N, N+1) gleichzeitig an, so kann mit dieser Redundanz die Genauigkeit und Verlässlichkeit der W(N) Messung noch erhöht werden. Widersprüchliche oder unlogische Messungen können bei gleichzeitiger Anwendung beider Verfahren durch den Mikrocontroller erfasst und entsprechend korrigiert werden.

Keine DC Gegenlichtquelle, aber versehentlicher Blick in die Eigenlichtquelle

Es kann im Extremfall angenommen werden, dass bei tief schwarzer Nacht und störungsfreier Sicht (z.B. völlig allein im Wald) gilt:

$$U \ (N) = 0$$

Daraus folgt mit obiger Formel (2) = A (N) = U (N) + W (N), dass gilt:

$$A \ (N) = W \ (N)$$

Die Brille kann in diesem Falle auch komplett offen/transparent sein, und der Scheinwerfer auch dauerhaft an bzw. scheinbar oder weitgehend dauerhaft an (z.B. vereinzelte Messpulse alle 300ms), so dass weiterhin die oben beschriebenen Delta-Messungen stattfinden können. Erst mit plötzlichem Auftreten von Störlicht wird die Brille automatisch wieder in den üblichen PWM-Modulationsmodus überführt.

Starke AC-Gegenlichtquelle, wie z.B. elektrische Kunstlichtquelle, die z.B. aus dem 50/60Hz Niederspannungsnetz gespeist wird

Der Aussensensor OS bzw. OL, OR verfügt über drei wesentliche Eigenschaften:

1) Er ist vergleichsweise wesentlich schneller als industrielles Kunstlicht (100 - 120 Hz) und kann dieses elektronisch auflösen und mittels Mikrokontroller problemlos erkennen.

2) Er ist zudem wie ein Messgerät genormt (kann Werte in Lux oder vergleichbaren Lichttechnischen Einheiten bzw. in zugehörigen Spannungsäquivalenten Werten ausgeben) und ist mit der menschlichen Augenempfindlichkeitskurve gewichtet, so dass er auch Lichtstärke messen kann.

3) Er ist vorzugsweise, aber nicht notwendigerweise, baugleich mit dem Innen-Sensor IS, so dass der Micro-Controller ohne aufwendige Umrechnung, sofort in Echtzeit "Vergleichsmessungen" zwischen Innen (durch das LCD hindurch) und Außen (am LCD vorbei) machen kann.

Gibt es nur eine einzelne dominante Kunstlichtquelle, so dass eine zyklische 100 / 120 Hz Schwingung vom Außen-Sensor detektiert werden kann, so legt dieser den Startzeitpunkt $T_{Null}$ der Grundfrequenz der PWM der Brille fest - sowie die Frequenz der PWM - so dass beide Systeme derart synchron laufen, dass das Helligkeitsmaximum der externen Lichtquelle immer genau am Anfang eines Zyklus steht und sofort vom Außen-Sensor OS und auch vom Innensensor IS gemessen werden kann. Der Innensensor IS kann diese maximale Helligkeit der Kunstlichtquelle ebenfalls messen, weil am Anfang eines Zyklus die Brille stets "offen" ist, d.h. die Flüssigkeitskristallzelle transparent ist. Somit messen der Außensensor OS und der Innen-sensor IS im Grunde das gleiche Licht, nur mit dem kleinen Unterschied, dass sich vor dem Innensensor IS das transparente LCD befindet, so dass IS etwas weniger Licht abbekommt - nämlich abzüglich der temperaturabhängigen und alterungsabhängigen Transmission im Durchlasszustand - z.B. 50% weniger bei gekreuzten Polfiltern (Polarisator-Analysator-Stellung).

Weiterhin sind die Innen- und Außen-Sensoren IS1 und OS1 auch räumlich sehr nah angeordnet, auf einer gedachten Achse z.B. maximal 3 mm auseinander liegend - auch "Messpärchen No.1" (MP1) genannt. Somit können auch Ortsfrequenzen OF (i.w.S. "Streifenmuster") von OF > 3 mm zu keinerlei Messfehler führen. Zudem existiert ein weiteres Messpärchen MP2 bestehend aus IS2 und OS2 jeweils orthogonal zum vorgenannten Messpärchen MP1, so dass Schachbrettmuster, also Ortsfrequenzen, die senkrecht zu vorgenannten Ortsfrequenzen verlaufen, entsprechend erfasst werden können, sofern diese mehr als 3 mm betragen. Beide Messpärchen (MP1 und MP2) liefern Werte, die vom Mikrocontroller derart ausgewertet werden können, dass "geometrische Mittelwerte" gemäß dem gedachten Dreieck zwischen Pupillen-Mittelpunkt-Stellung und Sensoranordnung gebildet werden können.

Integration binnen eines Zyklus

Der Innensensor IS misst das durch das LC auftreffende Licht und integriert dieses Licht im Rahmen einer Leerlauf-Initialisierungs-Phase im allerersten Zyklus von 100 oder 120 Hz in dem die Brille komplett offen bleibt (siehe Fig. 11). Da es nur ein einziger Zyklus aus einem synchronen 100 oder 120 Hz System ist (also von weiteren folgenden 109 oder 119 geregelten Zyklen), nimmt das menschliche Auge dies nicht wahr. Es liegt aber ein erstes Integrationsergebnis vom Zyklus T vor.

Bildet der Sensor IS dabei ein Integral über beispielsweise eine Konstante (DC Störlicht), so ergibt sich eine gerade aufsteigende Linie (siehe Fig.11), die nach Überschreiten einer Sollwert-Schwelle (Sollwert-Trigger) das komplette Schließen der Brille veranlasst (hartes on-off Keying via PWM). Dies hat zudem den Vorteil, dass noch binnen eines jeweiligen Zyklus T eine Entscheidung und Reaktion erfolgt, ohne dass man den T+1 oder T-1 oder sonstige weitere Zyklen mit einbeziehen muss, so wie es bei einer "analog mathematischen Berechnung in der Frequenzdomain eines PIDReglers" normalerweise nötig wäre. Es ist also keinerlei Fourier-Transformation nötig - weder FFT noch FT, DFT, usw.

Die sogenannte Regelung ist also in diesem Falle "hart" ausgelegt und reagiert in Echtzeit bereits im Zyklus T auf einen Sollwert - auch "mikroskopische Regelung" genannt.

Die sogenannte "Makroskopische Regelung":
Davon unbenommen kann aber dieser mikroskopische Integrationswert aus dem N-ten Zyklus in einem flüchtigen Zwischenspeicher abgelegt werden, so dass er für weitere nachfolgende Integrationswerte als "fließender/gleitender Mittelwert Korrekturwert", d.h. als Makroskopischer Integrationswert zur Verfügung steht - etwa binnen eines Viertels oder Drittels eines 100 Hz oder 120 Hz Zyklus (also binnen unmerklichen Sekundenbruchteilen).

Somit reagiert die Regelung im Falle von schwankendem Kunstlicht immer richtig. Fig. 11 zeigt hierzu die Initialisierungsphase mit noch unbekanntem Ausgang bzw. unbekannter Außenhelligkeit (Gewichtungsfaktor Beta), danach im Zyklus 2 gefolgt von einem auf 1 bzw. maximale Helligkeit und Modulationshub normierten Zyklus (Gewichtungsfaktor Alpha), in dem das Integral bei Erreichen eines Sollwertes Thres das Schließen der Brille veranlasst ($T_{OFF}$).

Im dritten Zyklus ist beispielsweise gezeigt, wie die Außenhelligkeit sich erhöht hat und zudem schwankt. Das entsprechende Integral (Graph in der Bildmitte) läuft nun steiler an, so dass der Sollwert Thres schneller erreicht wird und dementsprechend die Brille zeitlich früher schließt - $T_{OFF}$ ist also länger als im Zyklus zuvor. Die Integralwerte werden am Ende eines jeden Zyklus auf null gesetzt, so dass jeder Zyklus in Echtzeit in seiner Durchlässigkeit TR geregelt wird.

Szenario: Mehrere starke AC Gegenlichtquellen, wie z.B. elektrische Kunstlichtquellen, die z.B. aus diversen Netzen gespeist werden, so dass Frequenzgemische vorliegen.

Ein Gemisch von diversen sich überlagernden Frequenzen kann dazu führen, dass der Außensensor nicht mehr auf eine bestimmte Störfrequenz synchronisieren kann. Dies kann aber auch Vorteile haben, da sich ein Gemisch im Oszillogramm wie "Rauschen" darstellt, welches kaum mehr Täler und Aussetzer von Fremdlicht aufweist, da es aufgrund von Überlagerungen ein stabiles "Grundrauschen" gibt. In diesem Falle wird die Brille bzw. der Mikrocontroller den Versuch der Synchronisation abbrechen und einfach auf eine typische voreingestellte Arbeitsfrequenz schalten, z.B. auf 70 Hz, um dort unbeirrt zu arbeiten, gemäß obigem Integrationsschema.

Szenario: Mehrere starke gepulste Gegenlichtquellen, wie z.B. elektrische LED Kunstlichtquellen, z.B. wie das vorliegende oder ähnliche Systeme

Aufgrund der sofortigen Integration innerhalb eines Zyklus kann die Brille entsprechend zu machen, sobald ein Schwellwert erreicht ist. Da der Dynamikbereich und die Messgeschwindigkeit der Außen- und Innensensoren stets schneller und besser als das menschliche Auge sind, können auch extreme Intensitäten und schädliche Leistungen schützend abgewendet werden, wie z.B. extrem kurze Lichtpulse hoher Energie, wie sie z.B. von gepulsten Q-Switch-Lasern oder überpulsten LED stammen können.

Das menschliche Auge kann ab einer bestimmten anwachsenden Intensität bei gleichzeitig immer kürzer werdenden Pulsen dies nicht mehr vernünftig wahrnehmen und reagieren, so dass die Hornhaut und Netzhaut in Gefahr sind, Schaden zu nehmen.

Reaktion der Brille im Zweifelsfall:

Die Brille macht daher im Zweifelsfall bei hohen Intensitäten eher "zu" (Augenschutz) - während sie bei geringen Intensitäten, aber chaotischen, nicht näher interpretierbaren Frequenzmustern, auf die auch nicht synchronisiert werden kann, tendenziell eher "auf" gemacht wird, und versucht wird eine Art "mittlere Helligkeit" zu bestimmen, indem über mehrere Zyklen T integriert und gemittelt wird (z.B. über 300ms), so als wäre es Rauschen bzw. eine nahezu gleichförmige Quelle - während sie sich aber grundsätzlich im PWM Nachtsicht- und Dämmerungs-Betrieb befindet (5% bis ca. 20% offener PWM-Zeitschlitz mit entsprechend gepulstem Scheinwerfer).

Es wird also eine Brille vorgeschlagen. Die Brille hat ein Brillenglas mit einer Flüssigkristallzelle LC, deren Transmission TR zwischen transmittierend und blockierend umgeschaltet werden kann. Ferner weist die Brille einen Eye-Tracker ET auf, der die Blickrichtung des Auges bestimmen kann. Ferner mindestens einen Sensor IL, IR zum Messen der Helligkeit des auf ihn treffenden sichtbaren Lichts, wobei der Sensor augenseitig des Brillenglases angeordnet ist und die Helligkeit durch das mindestens eine Brillenglas ortsaufgelöst misst. Der Sensor kann die Helligkeit des aus der mit dem Eye-Tracker bestimmten Blickrichtung des Auges auf ihn treffenden sichtbaren Lichts bestimmen. Die Brille verfügt ferner über einen geschlossenen Regelkreis für die Regelung der Transmission der Flüssigkristallzelle, wobei ein Sollwert für die Helligkeit am Auge vorgegeben ist, und wobei der Regelkreis als Istwert die vom Sensor gemessene Helligkeit in Blickrichtung des Auges heranzieht.

zitierte Literatur

zitierte Patentliteratur

DE 10 2012 217 326 A1
DE 101 34 770 A1
DE 2 001 086 A,
EP 0 813 079 A2
US 2,066,680 A
US 5,172,256 A
WO 2013/143 998 A2

zitierte Nicht-Patentliteratur

Adrian, W. and Bhanji, A.: "Fundamentals of disability glare. A formula to describe stray light in the eye as a function of the glare angle and age." Proceedings of the First International Symposium on Glare, 1991, Orlando, Florida, pp. 185-194.

**EP 3 149 541 B1**

Douglas Mace, Philip Garvey, Richard J. Porter, Richard Schwab, Werner Adrian: "Countermeasures for Reducing the Effects of Headlight Glare"; Prepared for: The AAA Foundation for Traffic Safety, Washington, D.C., December 2001

Prof. Dr.-Ing. Gert Hauske: "Systemtheorie der visuellen Wahrnehmung", Teubner Verlag, Stuttgart, 1994

**Patentansprüche**

1. Brille für einen Träger mit mindestens einem Auge, mit

   mindestens einem Brillenglas;
   wobei das mindestens eine Brillenglas eine Flüssigkristallzelle (LC) aufweist, deren Transmission (TR) durch eine geeignete Ansteuerung veränderbar ist;
   einem Eye-Tracker (ET), der die Blickrichtung des Auges bestimmt;
   mindestens einem Sensor (IL, IR) zum Messen der Helligkeit des auf ihn treffenden sichtbaren Lichts;
   wobei der mindestens eine Sensor (IL, IR) augenseitig des Brillenglases angeordnet ist;
   wobei der mindestens eine Sensor (IL, IR) die Helligkeit durch das mindestens eine Brillenglas misst;
   wobei der mindestens eine Sensor (IL, IR)
   ein abbildendes System mit einer Kamera oder
   mindestens drei Sensoren, die ein Koordinatensystem aufspannen oder ein Facettenauge aufweist;
   wobei der mindestens eine Sensor (IL, IR) die Helligkeit des aus der mit dem Eye-Tracker (ET) bestimmten Blickrichtung des Auges auf ihn treffenden sichtbaren Lichts bestimmt; und mit
   einem geschlossenen Regelkreis (MC), der die Transmission der Flüssigkristallzelle (LC) regelt;
   wobei ein Sollwert für die Helligkeit am Auge vorgegeben ist;
   wobei der Regelkreis als Istwert die von dem mindestens einen Sensor (IL, IR) gemessene Helligkeit in Blickrichtung des Auges heranzieht.

2. Brille nach dem vorhergehenden Anspruch,
   **dadurch gekennzeichnet,**
   **dass** die Flüssigkristallzelle (LC) derart ausgebildet ist, dass sie ihre Transmission von 90% auf 10% und von 10% auf 90% in maximal 10 ms ändern kann.

3. Brille nach dem vorhergehenden Anspruch,
   **dadurch gekennzeichnet,**

   **dass** die Flüssigkristallzelle (LC) derart ausgebildet ist, dass die Transmission (TR) der Flüssigkristallzelle (LC) zwischen Zuständen hoher und niedriger Transmission umgeschaltet werden kann;
   **dass** Mittel zum Regeln oder Steuern der Zeiten des Zustands hoher ($T_{on}$) und niedriger ($T_{off}$) Transmission der Flüssigkristallzelle (LC) sowie des Wechsels zwischen diesen beiden Zuständen vorhanden sind;
   wobei die Regelung oder Steuerung und der geschlossene Regelkreises (MC) derart ausgebildet sind, dass die Zeiten des Zustands hoher Transmission ($T_{on}$) mit zunehmender Helligkeit des auf den mindestens einen Sensor (IL, IR) treffenden sichtbaren Lichts kürzer werden;
   wobei der Wechsel zwischen den Zuständen hoher ($T_{on}$) und niedriger ($T_{off}$) Transmission mit einer zeitlichen Frequenz stattfindet, die das menschliche Auge nicht auflösen kann.

4. Brille nach einem der vorhergehenden Ansprüche,
   **dadurch gekennzeichnet,**
   **dass** der Regelkreis derart ausgebildet ist, dass er bei der Bestimmung der Helligkeit aus der Blickrichtung des Auges eine nutzerspezifische Augen/Netzhaut-Empfindlichkeitskurve zur Gewichtung der Helligkeit berücksichtigen kann.

5. Brille nach einem der vorhergehenden Ansprüche,
   **dadurch gekennzeichnet,**
   **dass** der Sollwert des Regelkreises eine Helligkeit am Auge von 20 bis 400 lx vorgibt.

6. Brille nach einem der vorhergehenden Ansprüche,
   **gekennzeichnet durch**

mindestens einen weiteren Helligkeits-Sensor (OL, OR), der auf der vom Auge abgewandten Seite der Brille angeordnet ist (Außensensor) und die Helligkeit des Umgebungslichts bestimmt.

7. Brille nach dem vorhergehenden Anspruch,
**dadurch gekennzeichnet,**

**dass** der Sollwert des Regelkreises abhängig von der Helligkeit des Umgebungslichts verändert werden kann; und
**dass** die Veränderung des Sollwerts um mindestens einen Faktor 10 langsamer ist als die Regelung der Transmission der Flüssigkristalizelle.

8. Brille nach dem vorhergehenden Anspruch,
**dadurch gekennzeichnet,**
**dass** die Regelung derart ausgebildet ist, dass sie auf extreme Helligkeitswerte innerhalb von 10 μs bis einer Sekunde derart reagiert, dass die Flüssigkristallzelle (LC) auf den Zustand niedriger Transmission eingestellt wird.

9. Brille nach einem der vorhergehenden Ansprüche,
**gekennzeichnet durch**

zwei Brillengläser für zwei Augen eines Brillenträgers;
je einen augenseitigen Sensor für jedes Brillenglas zum Messen der Helligkeit des auf das jeweilige Auge treffenden sichtbaren Lichts; und durch
je einen Regelkreis für jedes Brillenglas.

10. Brille nach dem vorhergehenden Anspruch,
**dadurch gekennzeichnet,**
**dass** die Sollwerte für die beiden Augen um 1% bis 60% voneinander abweichen.

11. Brille nach Anspruch 9,
**dadurch gekennzeichnet,**
**dass** der Regelkreis (MC) derart ausgebildet ist, dass bei der Regelung der Helligkeit des auf ein Auge treffenden sichtbaren Lichts die Regelung der Helligkeit für das andere Auge berücksichtigt wird.

12. Brille nach Anspruch 1,
**gekennzeichnet durch**

mindestens eine Lichtquelle (S), die an der vom Auge abgewandten Seite der Brille angeordnet ist;
wobei die Lichtquelle abhängig von der Blickrichtung des Brillenträgers gesteuert wird.

13. Brille nach dem vorhergehenden Anspruch,
**gekennzeichnet durch**

Mittel zum Steuern oder Regeln der Leuchtzeiten und der Leuchtintensität der Lichtquelle (S) derart, dass diese während der Zeiten des Zustands hoher Transmission ($T_{on}$) der Flüssigkristallzelle (LC) leuchtet;
wobei das zeitliche Integral des Produkts aus
der Leuchtintensität der Lichtquelle (S) und
der Transmission (TR) der Flüssigkristallzelle (LC)
bei einer Veränderung der Zeiten des Zustands hoher Transmission ($T_{on}$) innerhalb einer vorgegebenen Toleranz konstant bleibt.

14. Brille nach Anspruch 1,
**gekennzeichnet durch**

- eine Lichtquelle zum Blenden eines Lebewesens, eines optischen Sensors oder einer Kamera, und/oder
- eine Anzeige an der vom Auge abgewandten Seite des Brillenglases und/oder
- eine Anzeige augenseitig des Brillenglases und/oder
- ein Head-Up-Display (HUD).

**15.** Verfahren zum Regeln der Helligkeit des auf mindestens ein Auge treffenden sichtbaren Lichts, mit folgenden Schritten:
eine Brille wird zur Verfügung gestellt, wobei die Brille folgendes aufweist:

mindestens ein Brillenglas;
wobei das mindestens eine Brillenglas eine Flüssigkristallzelle (LC) aufweist, deren Transmission (TR) durch eine geeignete Ansteuerung veränderbar ist;
einen Eye-Tracker (ET), der die Blickrichtung des Auges bestimmt;
mindestens ein Sensor (IL, IR) zum Messen der Helligkeit des auf den Sensor treffenden sichtbaren Lichts;
wobei der mindestens eine Sensor (IL, IR) augenseitig des Brillenglases angeordnet ist;
wobei der mindestens eine Sensor (IL, IR) die Helligkeit durch das mindestens eine Brillenglas misst;
wobei der mindestens eine Sensor (IL, IR)
ein abbildendes System mit einer Kamera oder
mindestens drei Sensoren, die ein Koordinatensystem aufspannen oder ein Facettenauge aufweist;
wobei der mindestens eine Sensor (IL, IR) die Helligkeit des aus der mit dem Eye-Tracker (ET) bestimmten Blickrichtung des Auges auf ihn treffenden sichtbaren Lichts bestimmt;
die Transmission der Flüssigkristallzelle (LC) wird mittels eines geschlossenen Regelkreises (MC) geregelt;
wobei ein Sollwert für die Helligkeit am Auge vorgegeben ist;
wobei der Regelkreis als Istwert die vom Sensor gemessene Helligkeit in Blickrichtung des Auges heranzieht.

**Claims**

**1.** Spectacles for a wearer with at least one eye, with

at least one spectacle lens;
wherein the at least one spectacle lens comprises a liquid crystal cell (LC), the transmission (TR) of which may be varied by a suitable control;
an eye tracker (ET) which determines the viewing direction of the eye;
at least one sensor (IL, IR) for measuring the brightness of the visible light incident thereon;
wherein the at least one sensor (IL, IR) is arranged on the eye-side of the spectacle lens;
wherein the at least one sensor (IL, IR) measures the brightness through the at least one spectacle lens;
wherein the at least one sensor (IL, IR) comprises
an imaging system with a camera or
at least three sensors that span a coordinate system or
a compound eye;
wherein the at least one sensor (IL, IR) determines the brightness of the visible light which is incident upon it from the viewing direction of the eye determined by the eye tracker (ET); and
a closed-loop control circuit (MC) to control the transmission of the liquid crystal cell (LC);
wherein a setpoint value for the brightness at the eye is preset;
wherein the control circuit takes the brightness measured by the at least one sensor (IL, IR) in the viewing direction of the eye as the actual value.

**2.** Spectacles according to the preceding claim,
**characterized in that**
the liquid crystal cell (LC) is so designed that it can change its transmission from 90% to 10% and from 10% to 90% in a maximum of 10 ms.

**3.** Spectacles according to the preceding claim,
**characterized in that**

the liquid crystal cell (LC) is so designed that the transmission (TR) of the liquid crystal cell (LC) may be switched between states of high and low transmission;
means are provided for controlling or regulating the times of the state of high ($T_{on}$) and low ($T_{off}$) transmission of the liquid crystal cell (LC) as well as the change between these two states;
wherein the control or regulation and the closed-loop control circuit (MC) are so designed that the times of the state of high transmission ($T_{on}$) become shorter with increasing brightness of the visible light incident on the at least one sensor (IL, IR);

wherein the change between the states of high ($T_{on}$) and low ($T_{off}$) transmission takes place with a temporal frequency which the human eye cannot resolve.

4. Spectacles according to one of the preceding claims,
   **characterized in that**
   the control circuit is so designed that, when determining the brightness from the viewing direction of the eye, it can take into account a user-specific eye/retina sensitivity curve for weighting the brightness.

5. Spectacles according to one of the preceding claims,
   **characterized in that**
   the setpoint value of the control circuit prescribes a brightness at the eye of 20 to 400 lx.

6. Spectacles according to one of the preceding claims,
   **characterized in that**
   at least one further brightness sensor (OL, OR) is arranged on the side of the spectacles facing away from the eye (external sensor) and determines the brightness of the ambient light.

7. Spectacles according to the preceding claim,
   **characterized in that**

   the setpoint value of the control circuit may be changed as a function of the brightness of the ambient light; and the change in the setpoint value is slower than the control of the transmission of the liquid crystal cell by a factor of at least 10.

8. Spectacles according to the preceding claim,
   **characterized in that**
   the control is so designed that it reacts to extreme brightness values within 10 $\mu$s to one second such that the liquid crystal cell (LC) is set to the state of low transmission.

9. Spectacles according to one of the preceding claims,
   **characterized by**

   two spectacle lenses for two eyes of a spectacle wearer;
   an eye-side sensor for each spectacle lens for measuring the brightness of the visible light incident on the respective eye; and
   a control circuit for each spectacle lens.

10. Spectacles according the preceding claim,
    **characterized in that**
    the setpoint values for the two eyes differ from one another by 1% to 60%.

11. Spectacles according to claim 9,
    **characterized in that**
    the control circuit (MC) is so designed that when regulating the brightness of the visible light incident on an eye the regulation of the brightness for the other eye is taken into account.

12. Spectacles according to claim 1,
    **characterized by**

    at least one light source (S) arranged on the side of the spectacles facing away from the eye;
    wherein the light source is controlled as a function of the viewing direction of the spectacle wearer.

13. Spectacles according to the preceding claim,
    **characterized by**

    means for controlling the lighting times and the luminous intensity of the light source (S) such that it illuminates during the times of the state of high transmission ($T_{on}$) of the liquid crystal cell (LC);
    wherein the temporal integral of the product of

the luminous intensity of the light source (S) and
the transmission (TR) of the liquid crystal cell (LC)
remains constant within a predetermined tolerance upon a change in the times of the state of high transmission ($T_{on}$).

14. Spectacles according to claim 1,
**characterized by**

- a light source for the dazzling of a living being, an optical sensor or a camera, and/or
- a display on the side of the spectacle lens facing away from the eye, and/or
- a display on the eye-side of the spectacle lens, and/or
- a head-up display (HUD).

15. Method for regulating the brightness of the visible light incident on at least one eye comprising the following steps:
a pair of spectacles is provided, wherein the spectacles comprise:

at least one spectacle lens;
wherein the at least one spectacle lens has a liquid crystal cell (LC), the transmission (TR) of which may be varied by a suitable control;
an eye tracker (ET) which determines the viewing direction of the eye;
at least one sensor (IL, IR) for measuring the brightness of the visible light incident on the sensor;
wherein the at least one sensor (IL, IR) is arranged on the eye-side of the spectacle lens;
wherein the at least one sensor (IL, IR) measures the brightness through the at least one spectacle lens;
wherein the at least one sensor (IL, IR) comprises
an imaging system with a camera or
at least three sensors which span a coordinate system or
a compound eye;
wherein the at least one sensor (IL, IR) determines the brightness of the visible light from the viewing direction of the eye determined by the eye tracker (ET);
the transmission of the liquid crystal cell (LC) is regulated by a closed-loop control circuit (MC);
wherein a setpoint value for the brightness at the eye is preset;
wherein the control loop takes the brightness measured by the sensor in the viewing direction of the eye as the actual value.

**Revendications**

1. Lunettes pour un porteur ayant au moins un oeil, avec

au moins un verre de lunettes ;
dans lequel ledit au moins un verre de lunettes comprend une cellule à cristaux liquides (LC) dont la transmission (TR) peut être modifiée par une commande appropriée ;
un eye-tracker (ET) qui détermine la direction du regard de l'oeil ;
au moins un capteur (IL, IR) pour mesurer la luminosité de la lumière visible qui le frappe ;
dans lequel ledit au moins un capteur (IL, IR) est disposé du côté de l'œil du verre de lunettes ;
dans lequel ledit au moins un capteur (IL, IR) mesure la luminosité à travers ledit au moins un verre de lunettes ;
dans lequel le au moins un capteur (IL, IR) comprend
un système d'imagerie comprenant une caméra ou
au moins trois capteurs couvrant un système de coordonnées ou
un oeil à facettes
le au moins un capteur (IL, IR) déterminant la luminosité de la lumière visible qui le frappe à partir de la direction du regard de l'oeil déterminée avec l'eye-tracker (ET) ; et avec
dans lequel ledit au moins un capteur (IL, IR) détermine la luminosité de la lumière visible qui le frappe à partir de la direction du regard de l'oeil déterminée avec l'eye-tracker (ET) ; et avec
un circuit de régulation fermé (MC) qui régule la transmission de la cellule à cristaux liquides (LC) ;
dans lequel une valeur de consigne pour la luminosité au niveau de l'œil est prédéfinie ;
dans lequel le circuit de régulation utilise comme valeur réelle la luminosité mesurée par l'au moins un capteur (IL, IR) dans la direction du regard de l'œil.

**2.** Lunettes selon la revendication précédente, **caractérisé**

**en ce que** la cellule à cristaux liquides (LC) est conçue de manière à pouvoir modifier sa transmission de 90% à 10% et de 10% à 90% en 10 ms au maximum.

**3.** Lunettes selon la revendication précédente, **caractérisé**

**en ce que** la cellule à cristaux liquides (LC) est conçue de telle sorte que la transmission (TR) de la cellule à cristaux liquides (LC) peut être commutée entre des états de transmission élevée et de transmission faible ;

**en ce qu'**il existe des moyens de régulation ou de commande des temps de l'état de transmission élevée (Ton) et de l'état de transmission faible (Toff) de la cellule à cristaux liquides (LC) ainsi que du passage entre ces deux états ;

dans lequel la régulation ou la commande et la boucle de régulation fermée (MC) sont conçues de telle sorte que les temps de l'état de transmission élevée (Ton) deviennent plus courts à mesure que la luminosité de la lumière visible atteignant l'au moins un capteur (IL, IR) augmente ;

dans lequel le changement entre les états de transmission élevée (Ton) et faible (Toff) se produit à une fréquence temporelle que l'œil humain ne peut pas résoudre.

**4.** Lunettes selon l'une quelconque des revendications précédentes, **caractérisé**

**en ce que** le circuit de régulation est conçu de telle sorte que, lors de la détermination de la luminosité à partir de la direction du regard de l'œil, il peut prendre en compte une courbe de sensibilité oeil/rétine spécifique à l'utilisateur pour pondérer la luminosité.

**5.** Lunettes selon l'une des revendications précédentes, **caractérisé**

**en ce que** la valeur de consigne du circuit de régulation définit une luminosité au niveau de l'œil de 20 à 400 lx.

**6.** Lunettes selon l'une des revendications précédentes, **caractérisé par**

au moins un autre capteur de luminosité (OL, OR), qui est disposé sur le côté des lunettes opposé à l'œil (capteur extérieur) et qui détermine la luminosité de la lumière ambiante.

**7.** Lunettes selon la revendication précédente, **caractérisé**

**en ce que** la valeur de consigne de la boucle de régulation peut être modifiée en fonction de la luminosité de la lumière ambiante ; et

**en ce que** la modification de la valeur de consigne est plus lente d'au moins un facteur 10 que la régulation de la transmission de la cellule à cristaux liquides.

**8.** Lunettes selon la revendication précédente, **caractérisé**

**en ce que** la commande est conçue pour réagir à des valeurs de luminosité extrêmes en l'espace de 10 µs à une seconde de telle sorte que la cellule à cristaux liquides (LC) soit réglée sur l'état de faible transmission.

**9.** Lunettes selon l'une des revendications précédentes, **caractérisé par**

deux verres de lunettes pour deux yeux d'un porteur de lunettes ;

un capteur côté oeil pour chaque verre de lunettes pour mesurer la luminosité de la lumière visible arrivant sur l'œil correspondant ; et par

un circuit de contrôle pour chaque verre de lunettes.

**10.** Lunettes selon la revendication précédente, **caractérisé**

**en ce que** les valeurs de consigne pour les deux yeux diffèrent de 1% à 60%.

**11.** Lunettes selon la revendication 9,
**caractérisé**
**en ce que** le circuit de régulation (MC) est conçu de telle sorte que, lors de la régulation de la luminosité de la lumière visible arrivant sur un oeil, la régulation de la luminosité pour l'autre oeil est prise en compte.

**12.** Lunettes selon la revendication 1,
**caractérisé par**

au moins une source lumineuse (S), qui est disposée sur le côté des lunettes opposé à l'œil ;
dans lequel la source lumineuse est commandée en fonction de la direction du regard du porteur de lunettes.

**13.** Lunettes selon la revendication précédente,
**caractérisé par**

des moyens de commande ou de régulation des temps d'éclairage et de l'intensité lumineuse de la source lumineuse (S) de manière à ce qu'elle éclaire pendant les temps de l'état de transmission élevée (Ton) de la cellule à cristaux liquides (LC) ;
dans lequel l'intégrale temporelle du produit de
l'intensité lumineuse de la source de lumière (S) et
de la transmission (TR) de la cellule à cristaux liquides (LC)
reste constante lors d'une variation des temps de l'état de transmission élevée (Ton) dans une tolérance prédéterminée.

**14.** Lunettes selon la revendication 1,
**caractérisé par**

- une source lumineuse pour éblouir un être vivant, un capteur optique ou une caméra, et/ou
- un affichage sur le côté du verre de lunettes opposé à l'œil et/ou
- un affichage du côté de l'œil du verre de lunettes et/ou
- un affichage tête haute (HUD).

**15.** Procédé de réglage de la luminosité de la lumière visible frappant au moins un oeil, comprenant les étapes suivantes :
une paire de lunettes est fournie, dans laquelle les lunettes comprennent :

au moins un verre de lunettes ;
dans lequel ledit au moins un verre de lunettes comprend une cellule à cristaux liquides (LC) dont la transmission (TR) peut être modifiée par une commande appropriée ;
un eye-tracker (ET) qui détermine la direction du regard de l'œil ;
au moins un capteur (IL, IR) pour mesurer la luminosité de la lumière visible frappant le capteur ;
dans lequel ledit au moins un capteur (IL, IR) est disposé du côté de l'œil du verre de lunettes ;
dans lequel ledit au moins un capteur (IL, IR) mesure la luminosité à travers ledit au moins un verre de lunettes ;
dans lequel ledit au moins un capteur (IL, IR) comprend
un système d'imagerie comprenant une caméra ou
au moins trois capteurs couvrant un système de coordonnées ou
un oeil à facettes ;
dans lequel ledit au moins un capteur (IL, IR) détermine la luminosité de la lumière visible qui le frappe à partir de la direction du regard de l'œil déterminée avec l'eye-tracker (ET) ;
la transmission de la cellule à cristaux liquides (LC) est réglée au moyen d'une boucle de régulation fermée (MC) ;
dans lequel une valeur de consigne pour la luminosité au niveau de l'œil est prédéfinie ;
dans lequel le circuit de régulation utilise comme valeur réelle la luminosité mesurée par le capteur dans la direction du regard de l'œil.

Fig. 1

Fig. 2

EP 3 149 541 B1

Fig. 3

Fig. 4

Fig. 5

Fig. 5B

Fig. 6

Fig. 7

EP 3 149 541 B1

Fig. 8

Fig. 9

Fig. 10

Fig. 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5172256 A
- DE 102012217326 A1
- US 2066680 A
- DE 10134770 A1
- DE 2001086 A
- WO 2013143998 A2
- EP 0813079 A2
- DE 102014107587

**In der Beschreibung aufgeführte Nicht-Patentliteratur**

- **DOUGLAS MACE ; PHILIP GARVEY ; RICHARD J. PORTER ; RICHARD SCHWAB ; WERNER ADRIAN.** Counter-measures for Reducing the Effects of Headlight Glare. *Prepared for: The AAA Foundation for Traffic Safety, Washington, D.C.,* Dezember 2001
- **ADRIAN, W. ; BHANJI, A.** Fundamentals of disability glare. A formula to describe stray light in the eye as a function of the glare angle and age. *Proceedings of the First International Symposium on Glare, Orlando, Florida,* 1991, 185-194
- **ADRIAN, W. ; BHANJI, A.** Fundamentals of disability glare. A formula to describe stray light in the eye as a function of the glare angle and age. *Proceedings of the First International Symposium on Glare,* 1991, 185-194
- **DOUGLAS MACE ; PHILIP GARVEY ; RICHARD J. PORTER ; RICHARD SCHWAB ; WERNER ADRIAN.** Countermeasures for Reducing the Effects of Headlight Glare. *Prepared for: The AAA Foundation for Traffic Safety, Washington, D.C.,* Dezember 2001
- **PROF. DR.-ING. GERT HAUSKE.** Systemtheorie der visuellen Wahrnehmung. Teubner Verlag, 1994